# EUROPEAN PATENT SPECIFICATION

(11) **EP 4 086 333 B1**
(45) Date of publication and mention of the grant of the patent: **28.05.2025**
(21) Application number: 21920108.4
(22) Date of filing: 05.08.2021
(51) Int. Cl.: C12M 1/34, C12M 1/36, C12M 1/00, C12Q 1/686, B67B 3/20, B67B 7/15, B04B 5/04, G01N 35/00, G01N 35/04, G01N 35/10, B67B 3/00, B67B 7/02

(54) **FULLY AUTOMATIC GENE ANALYSIS APPARATUS AND GENE ANALYSIS METHOD**
VOLLAUTOMATISCHE GENANALYSEVORRICHTUNG UND GENANALYSEVERFAHREN
APPAREIL D'ANALYSE GÉNÉTIQUE ENTIÈREMENT AUTOMATIQUE ET PROCÉDÉ D'ANALYSE GÉNÉTIQUE

(30) Priority: 17.03.2021 CN 202110286476; 17.03.2021 CN 202110288381
(43) Date of publication of application: 09.11.2022
(73) Proprietor: HANGZHOU BIOER TECHNOLOGY CO., LTD., Hangzhou, Zhejiang 310000 (CN)
(72) Inventor: LI, Dong, Hangzhou Zhejiang 310000 (CN); CAO, Jintao, Hangzhou Zhejiang 310000 (CN); LI, Zhihai, Hangzhou Zhejiang 310000 (CN); ZHANG, Kang, Hangzhou Zhejiang 310000 (CN); ZHANG, Leijian, Hangzhou Zhejiang 310000 (CN); YU, Yue, Hangzhou Zhejiang 310000 (CN); HE, Xianhan, Hangzhou Zhejiang 310000 (CN)
(74) Representative: Münchow, Vera Ute Barbara
(86) International application number: PCT/CN2021/111017
(87) International publication number: WO 2022/193532

(56) References cited:
- CN-A- 108 949 548
- CN-A- 113 046 224
- CN-A- 113 072 018
- US-A1- 2009 117 620
- US-A1- 2019 048 411
- TOSHIYUKI IKEDA ET AL: "TOTAL CLINICAL LABORATORY TESTING SYSTEM FOR LABORATORY AUTOMATION", HITACHI REVIEW, HITACHI LTD. TOKYO, JP, vol. 41, no. 4, 1 September 1992 (1992-09-01), pages 167 - 172, XP000334656, ISSN: 0018-277X

## Description

### Cross-reference to Related Applications

The present disclosure claims priority to Chinese Patent Application No. 2021102864766, entitled "Full-automatic Gene Analysis Apparatus and Gene Analysis Method", filed with National Intellectual Property Administration of P.R. China on March 17, 2021, and Chinese Patent Application No. 2021102883818, entitled "Molecular Detection Centrifugation Lid Opening and Closing Device", filed with National Intellectual Property Administration of P.R. China on March 17, 2021.

### Technical Field

The present disclosure relates to the field of medical detection technologies, and particularly to a full-automatic gene analysis apparatus and a gene analysis method.

### Background Art

During gene detection, such as PCR detection, or the like, an operator is required to utilize a detection apparatus for detection in a complete laboratory, thus requiring a large detection space; the detection apparatus cannot realize full automatic detection, and a whole detection process requires a large amount of manual intervention, such that an operation process is cumbersome, a detection efficiency is low, and the operator is liable to operate incorrectly to influence a detection result.

In addition, during nucleic acid extraction in the gene detection process, currently, most working operations of opening and closing lids of PCR tubes are completed manually, if the lid is opened manually, aerosol may be generated due to a pressure difference between the interior and the exterior of the test tube during opening the lid, and if the operator holds the test tube directly with the hand to open the lid, an infection risk is increased, and meanwhile, a possibility of cross contamination among the test tubes is increased due to the direct hand operation of the operator; moreover, a manual lid opening efficiency is extremely low, and manual labor intensity is great. Currently, a device capable of automatically opening and closing the lid appears on the market to replace manual labor.

Full automatic analysis apparatuses are described in US 2019/048411 A1 (referring to a nucleic acid analyzer), by Toshiyuki Ikeda et al. in "Total clinical laboratory testing system for laboratory automation", Hitachi Review, Hitachi Ltd., Tokyo, Japan, vol. 41., n. 4, 1 September 1992, pages 167-172, ISSN: 0018-277X for clinical chemistry and immunology testing, and US 2009/0117620 A1, the latter forming the basis for the preamble of claim 1.

However, the existing test tube lid opening and closing device can only achieve automatic lid opening and closing function which is single, and cannot complete operations after the lid is opened and closed.

### Summary

Furthermore, the present disclosure provides a full-automatic gene analysis apparatus to solve problems that an existing detection apparatus requires a large detection space, wastes labor and has a low detection efficiency.

The present disclosure provides a full-automatic gene analysis apparatus which has a specific technical solution as defined in claim 1.

Optionally, the PCR buffer region may be provided across the nucleic acid purification region and the PCR detection region, the PCR buffer region may be a sealed chamber, and the sealed chamber may be provided with a first opening located in the nucleic acid purification region and a second opening located in the PCR detection region; the first opening may be provided with a first door for opening and closing the first opening, and the second opening may be provided with a second door for opening and closing the second opening; the centrifugal transfer device and the second lid opening and closing device may be both located within the sealed chamber and may be located below the first opening and the second opening.

Optionally, the full-automatic gene analysis apparatus may further include a negative pressure suction device and an air treatment device, the negative pressure suction device may be respectively communicated with the PCR detection region and the air treatment device, the air treatment device may be communicated with outside atmosphere. The negative pressure suction device is configured to provide a negative pressure effect such that a gas pressure of the nucleic acid purification region is greater than that of the PCR buffer region, and a gas pressure of the PCR buffer region is greater than that of the PCR detection region.

Optionally, the air treatment device may include an air filter, a reagent solution filter and an exhaust pipe, and the negative pressure suction device, the air filter, the reagent solution filter and the exhaust pipe may be communicated sequentially.

Optionally, a one-way valve may be provided between the sealed chamber and the PCR detection region for allowing a unidirectional gas stream flow from the sealed chamber to the PCR detection region.

Optionally, the first taking and placing device may include a motion mechanism and a first clamping mechanism connected to each other, and the motion mechanism may be configured to drive the first clamping mechanism to translate and lift and drop; the first clamping mechanism may include two clamping arms, each clamping arm may be provided with a first clamping structure and a second clamping structure, the two first clamping structures are fitted to form a first clamping portion, and the two second clamping structures are fitted to form a second clamping portion.

Optionally, the first clamping structure may be an arc groove formed in an inner side of the clamping arm, and/or the second clamping structure may be a clamping block provided at a lower end of the clamping arm.

Optionally, the first taking and placing device may further include a pipetting device configured to perform a pipetting operation after a pipette tip is taken.

Optionally, the PCR detection device may include a plurality of PCR detection mechanisms, the plurality of PCR detection mechanisms may be sequentially arranged in a preset direction, the second taking and placing device may be configured to sequentially place the PCR tubes into the plurality of PCR detection mechanisms. The full-automatic gene analysis apparatus is configured for performing detection after all hole positions of the PCR detection mechanisms have been loaded with the PCR tubes.

In an optional embodiment, the centrifugal transfer device may further include a tube body fixing assembly; a motor base plate may be provided at a bottom of the rotary driving member, a fixing base plate may be provided on the side of the motor base plate away from the rotary driving member, and the second lid opening and closing device and the tube body fixing assembly may be both provided on the fixing base plate; the tube body fixing assembly has a tube body clamp for clamping the PCR tube.

In an optional embodiment, the tube body fixing assembly may include a clamping driving member, a clamping screw and a clamping nut; the clamping driving member may be in transmission connection with the clamping screw, the clamping nut may be provided on the clamping screw, the clamping nut is connected with the tube body clamp, and the clamping driving member may be configured to drive the clamping screw to rotate in an axis direction of the clamping screw, so as to move the tube body clamp towards or away from the PCR tube.

In an optional embodiment, the tube body fixing assembly may further include a clamping mounting frame; the clamping driving member is connected with the clamping mounting frame, and the clamping mounting frame may be provided with a limiting signal sensor and an origin signal sensor.

In an optional embodiment, the centrifugal transfer device may further include an outer housing, an upper cover plate and a rear cover plate; the upper cover plate and the rear cover plate may be both connected with the outer housing, the fixing base plate is respectively connected with the outer housing and the rear cover plate, the fixing base plate, the upper cover plate, the rear cover plate and the outer housing form an accommodating cavity, and the second lid opening and closing device may be located in the accommodating cavity.

In an optional embodiment, an upper cover sealing strip may be provided between the outer housing and the upper cover plate; the fixing base plate may be provided with a lower sealing strip; a first sealing strip may be provided between the rear cover plate and the outer housing; an inner frame may be provided in the accommodating cavity, and a second sealing strip may be provided between the inner frame and the outer housing.

In an optional embodiment, the centrifugal transfer device may further include a door opening and closing assembly; the upper cover plate may be provided with an opening, the door opening and closing assembly may have a door pressing plate, the opening may be plugged by the door pressing plate, and a door-plate sealing strip is provided between the door opening and closing assembly and the upper cover plate.

In an optional embodiment, the door opening and closing assembly may include a linear motor, a door frame, a first spring hanger, a second spring hanger and an elastic member; the linear motor may be provided on the upper cover plate, the linear motor is in transmission connection with the door frame, the first spring hanger may be provided on the door frame, a kidney shape hole may be provided in the door frame, and the door pressing plate extends into the kidney shape hole through the second spring hanger; one end of the elastic member may be connected with the first spring hanger, the other end of the elastic member may be connected with the second spring hanger, and the elastic member may be configured to give the door pressing plate a tendency to move towards the door frame.

In an optional embodiment, the upper cover plate may be provided with a limiting block; the linear motor drives the door frame to move towards the limiting block, such that the door pressing plate abuts against the limiting block, and the door pressing plate moves along the kidney shape hole to tightly press the door-plate sealing strip.

The centrifugal transfer device in the present disclosure may include: the rotary disc, the second lid opening and closing device and the rotary driving member; the rotary disc is configured to mount the PCR tube, the second lid opening and closing device has the grabbing portion, and the grabbing portion can drive the tube lid on the PCR tube to move; the rotary driving member is in transmission connection with the rotary disc, and the rotary driving member has the rotary mode and the centrifugal mode; when in the rotary mode, the rotary driving member drives the PCR tube to move to the position below the grabbing portion; when in the centrifugal mode, the rotary driving member drives the PCR tube to perform centrifugal motion. The PCR tube is placed on the rotary disc, the rotary mode of the rotary driving member is started, the PCR tube is moved to the position below the grabbing portion, and the tube lid on the PCR tube is driven to move by the grabbing portion of the second lid opening and closing device, so as to complete a lid opening or closing operation; the centrifugal mode of the rotary driving member is started, and the rotary disc rotates at a high speed to drive the PCR tube to perform centrifugal motion, thus facilitating a centrifugal operation of reagents in the PCR tube; the lid opening and closing functions can be achieved, the reagents may be centrifuged, more functions are achieved, and the technical problem that in a prior art, an existing PCR tube lid opening and closing device can only achieve the single automatic lid opening and closing functions is solved.

In addition, the present disclosure further provides a gene analysis method in which the above apparatus is applied, including the following steps:
S1-performing nucleic acid extraction, wherein the first taking and placing device may transfer the sample tube on the sample rack to the first lid opening and closing device, so as to open a lid of the sample tube by the first lid opening and closing device, and then, a first pipette tip on the consumable rack is taken such that the first pipette tip sucks a sample in the sample tube, and places the sample sucked by the first pipette tip in the nucleic acid purification module for nucleic acid extraction;
S2-performing PCR system construction, wherein the first taking and placing device may transfer the PCR tube on the consumable rack to the centrifugal transfer device, a PCR tube lid is opened by the second lid opening and closing device, and the first taking and placing device then takes a second pipette tip on the consumable rack, allows the second pipette tip to suck a reagent, adds the reagent into the PCR tube, then takes a third pipette tip on the consumable rack, allows the third pipette tip to suck a gene sample extracted in the nucleic acid purification module, and adds the gene sample into the PCR tube;
S3-performing centrifugal treatment, wherein the PCR tube lid is closed by the second lid opening and closing device and then, the centrifugal transfer device performs rotary centrifugal treatment on the PCR tube and transfers the PCR tube after the centrifugal treatment to the PCR detection region; and
S4-performing PCR detection, wherein the second taking and placing device may transfer the PCR tube located in the PCR detection region to the PCR detection device for detection.

In the full-automatic gene analysis apparatus and the gene analysis method according to the present disclosure, the first taking and placing device is configured to take and place the sample tube or the PCR tube or perform the pipetting operation, the lid is automatically opened and closed by the lid opening and closing device, and then, the genes may be purified and extracted automatically, thus automatically constructing a PCR system; automatic transfer and centrifuging operations of the PCR tube are realized by the centrifugal transfer device, thus achieving plural purposes with one device; the PCR detection step is automatically performed by the second taking and placing device and the PCR detection device, and manual intervention is avoided in the whole process, thus improving a detection efficiency, guaranteeing detection accuracy, simplifying a required experimental apparatus, and reducing the space for detection.

### Brief Description of Drawings

To describe the technical solutions in the specific embodiments of the present disclosure or the prior art more clearly, the following briefly describes the accompanying drawings required for describing the specific embodiments or the prior art.
FIG. 1 is a top view of a full-automatic gene analysis apparatus according to an exemplary embodiment of the present disclosure;
FIG. 2 is a side view of the full-automatic gene analysis apparatus according to the exemplary embodiment of the present disclosure;
FIG. 3 is a first schematic partial structural diagram of the full-automatic gene analysis apparatus according to the exemplary embodiment of the present disclosure;
FIG. 4 is a second schematic partial structural diagram of the full-automatic gene analysis apparatus according to the exemplary embodiment of the present disclosure;
FIG. 5 is a schematic structural diagram of a centrifugal transfer device and a part of a sealed chamber of the full-automatic gene analysis apparatus according to the exemplary embodiment of the present disclosure;
FIG. 6 is a schematic structural diagram of the centrifugal transfer device and a second lid opening and closing device of the full-automatic gene analysis apparatus according to the exemplary embodiment of the present disclosure;
FIG. 7 is a schematic overall structural diagram of the centrifugal transfer device in the exemplary embodiment of the present disclosure;
FIG. 8 is a schematic structural diagram of the centrifugal transfer device in the exemplary embodiment of the present disclosure from a first perspective;
FIG. 9 is a schematic mounting structure diagram of a rotary driving member in the centrifugal transfer device in the exemplary embodiment of the present disclosure;
FIG. 10 is a schematic mounting structure diagram of a hanging basket in the centrifugal transfer device in the exemplary embodiment of the present disclosure;
FIG. 11 is a sectional structural view of a lid opening and closing assembly in the centrifugal transfer device in the exemplary embodiment of the present disclosure;
FIG. 12 is a schematic structural diagram of the lid opening and closing assembly in the centrifugal transfer device in the exemplary embodiment of the present disclosure;
FIG. 13 is a schematic structural diagram of a tube body fixing assembly in the centrifugal transfer device in the exemplary embodiment of the present disclosure;
FIG. 14 is a schematic structural diagram of a fixing seat in the centrifugal transfer device in the exemplary embodiment of the present disclosure;
FIG. 15 is a schematic exploded structural diagram of the centrifugal transfer device with an outer housing in the exemplary embodiment of the present disclosure;
FIG. 16 is a schematic structural diagram of the centrifugal transfer device with the outer housing in the exemplary embodiment of the present disclosure;
FIG. 17 is a schematic structural diagram of a door opening and closing assembly in the centrifugal transfer device in the present disclosure;
FIG. 18 is a schematic enlarged structural diagram of the door opening and closing assembly in the centrifugal transfer device in the present disclosure;
FIG. 19 is a schematic structural diagram of a first taking and placing device of the full-automatic gene analysis apparatus according to the exemplary embodiment of the present disclosure;
FIG. 20 is a schematic structural diagram of a first clamping mechanism of the full-automatic gene analysis apparatus according to the present disclosure;
FIG. 21 is a schematic structural diagram of a consumable rack of the full-automatic gene analysis apparatus according to the present disclosure; and
FIG. 22 is a schematic structural diagram of an air treatment device of the full-automatic gene analysis apparatus according to the present disclosure.

### Reference numerals:

100-body; 110-nucleic acid purification region; 120-PCR detection region; 130-PCR buffer region; 131-first opening; 132-second opening;
140-spare consumable placing region; 150-waste tip collecting region; 160-mounting chamber; 170-guide rail; 180-nucleic acid purification module;
200-first taking and placing device; 210-motion mechanism; 220-first clamping mechanism; 230-clamping arm; 231-first clamping structure; 232-second clamping structure;
300-first lid opening and closing device; 400-second lid opening and closing device; 500-sample rack; 600-consumable rack; 700-PCR detection device; 800-centrifugal transfer device; 810-rotary disc; 820-mounting assembly; 900-sample tube; 1000-PCR tube; 1100-negative pressure suction device; 1200-air treatment device; 1210-air filter; 1220-reagent solution filter; 1230-exhaust pipe;
3-rotary driving member; 6-tube lid placing portion; 7-grabbing portion; 8-gripper driving member; 9-connecting screw; 10-nut sleeve; 11-moving seat; 12-wedge; 13-lifting driving member; 14-fixing mounting plate; 15-fixing seat; 16-tube body fixing assembly; 17-motor base plate; 18-fixing base plate; 19-tube body clamp; 20-clamping driving member; 21-clamping screw; 22-clamping nut; 23-clamping mounting frame; 24-limiting signal sensor; 25-origin signal sensor; 26-outer housing; 27-upper cover plate; 28-rear cover plate; 29-upper cover sealing strip; 30-lower sealing strip; 31-first sealing strip; 32-inner frame; 33-second sealing strip; 34-door-plate sealing strip; 35-door opening and closing assembly; 36-door pressing plate; 37-linear motor; 38-door frame; 39-first spring hanger; 40-second spring hanger; 41-elastic member; 42-kidney shape hole; 43-limiting block.

### Detailed Description of Embodiments

To make the objectives, technical solutions and advantages of the exemplary embodiments of the present disclosure clearer, the technical solutions in the exemplary embodiments of the present disclosure are clearly and completely described with reference to the accompanying drawings in the exemplary embodiments of the present disclosure, and apparently, the described exemplary embodiments are not all but a part of the embodiments of the present disclosure. Generally, the assemblies of the exemplary embodiments of the present disclosure described and illustrated in the drawings herein may be arranged and designed in a variety of different configurations.

Accordingly, the following detailed description of the exemplary embodiments of the present disclosure provided in the drawings is not intended to limit the scope of protection of the present disclosure, but only represents selected exemplary embodiments of the present disclosure. All other exemplary embodiments obtained by a person of ordinary skill in the art based on the exemplary embodiments of the present disclosure without creative efforts shall fall within the protection scope of the present disclosure.

In descriptions of the present disclosure, it should be noted that, directions or positional relationships indicated by terms "center", "upper", "lower", "left", "right", "vertical", "horizontal", "inner", "outer", etc. are based on orientations or positional relationships shown in the accompanying drawings, or orientations or positional relationships of conventional placement of the product according to the present disclosure in use, and they are used only for describing the present disclosure and for description simplicity, but do not indicate or imply that an indicated device or element must have a specific orientation or be constructed and operated in a specific orientation. Therefore, it cannot be understood as a limitation on the present disclosure. In addition, the terms such as "first", "second", "third", or the like, are only used for distinguishing descriptions and are not intended to indicate or imply relative importance.

In addition, the terms of "horizontal", "vertical", and "overhung" and so on do not represent that the means is absolutely horizontal or overhung but it can be slightly tilted. For example, "horizontal" only means that the direction is more horizontal than "vertical" and can be slightly tilted, instead that this structure has to be horizontal completely.

In the description of the present disclosure, it still should be noted that unless specified or limited otherwise, the terms "provided", "mounted", "connected", and "coupled" and the like are used broadly, and may be, for example, fixed connections, detachable connections, or integral connections; may also be mechanical or electrical connections; may also be direct connections or indirect connections via intervening structures; may also be inner communications of two elements. The above terms can be understood by those skilled in the art according to specific situations.

Some embodiments of the present disclosure will be described in detail below with reference to the accompanying drawings. The embodiments described below and features therein may be combined with each other without conflicts.

Based on the description in the background art, the existing gene detection requires a large space, cannot realize full automatic detection, wastes labor, has a low detection efficiency, and requires an improvement of detection accuracy. In view of this, an exemplary embodiment of the present disclosure particularly proposes a full-automatic gene analysis apparatus intended to achieve full automation of gene analysis detection through linkage of various functional devices.

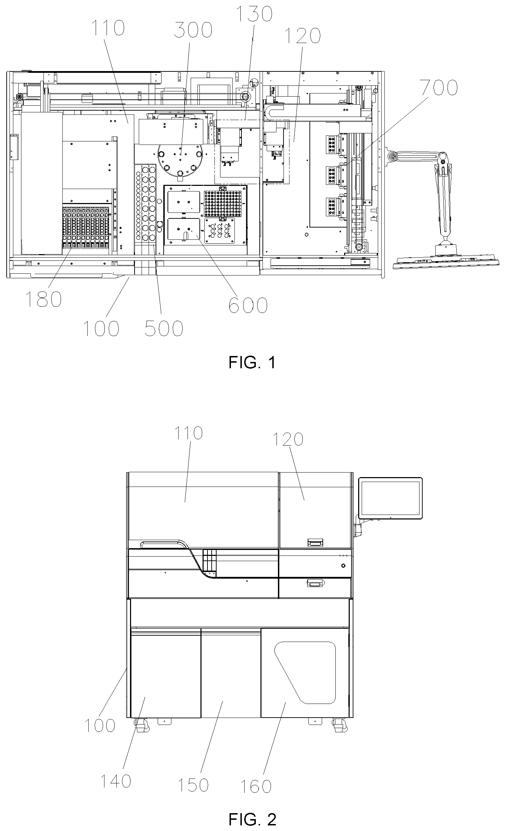

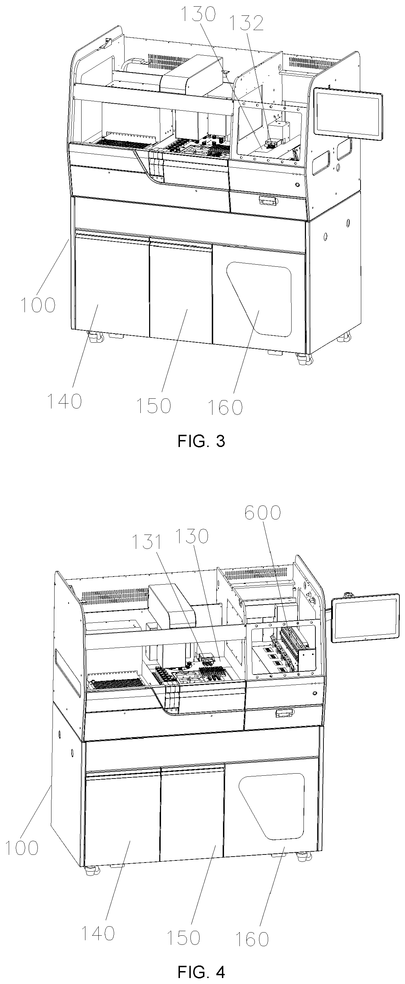

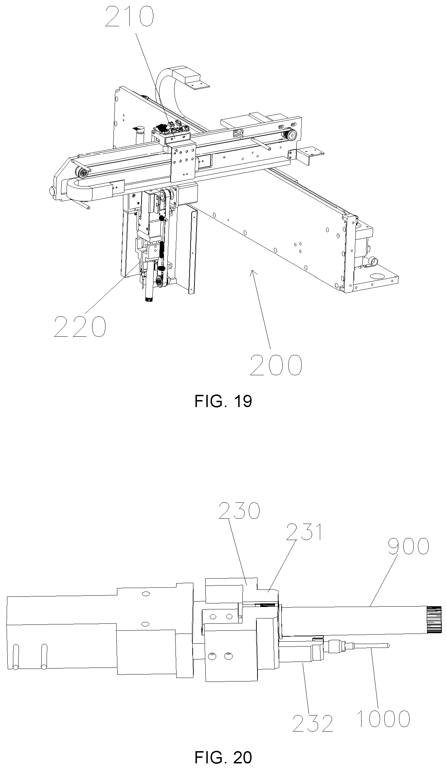

Referring to FIGS. 1, 2, 3 and 4, the full-automatic gene analysis apparatus according to the exemplary embodiment of the present disclosure may include a body 100, a first taking and placing device 200 (shown in FIG. 19), a second taking and placing device (not shown), a first lid opening and closing device 300, a second lid opening and closing device 400, a sample rack 500, a consumable rack 600, a PCR detection device 700, a centrifugal transfer device 800, and other functional devices, and it should be noted that FIG. 1 in the exemplary embodiment of the present disclosure only shows a part of an upper structure of the apparatus, and does not show the first taking and placing device 200 and other parts in the present embodiment.

The body 100 in the exemplary embodiment of the present disclosure may be used as a mounting base for other functional devices, and may have a structure similar to a multi-layer box in the drawing, or may be in other structural forms, and a bottom of the body 100 may be provided with a traveling mechanism, such as a roller, or the like, to facilitate movement of the whole apparatus. An upper portion of the body 100 in the exemplary embodiment of the present disclosure may be divided by a partition into a nucleic acid purification region 110 and a PCR detection region 120 isolated from each other, the body may be provided with a PCR buffer region spanning the nucleic acid purification region 110 and the PCR detection region 120, a nucleic acid purification module 180 and a template buffer module may be provided in the nucleic acid purification region 110 in the present embodiment, and the nucleic acid purification module 180 may be configured to place a nucleic acid extraction tool, such as a reagent strip (not shown), or the like. A lower portion of the body 100 in the exemplary embodiment of the present disclosure may be provided with a spare consumable placing region 140 and a waste tip collecting region 150, the spare consumable placing region 140 may be configured to place spare consumables, and the waste tip collecting region 150 may be configured to store waste pipette tips used in a detection process.

The first taking and placing device 200, the first lid opening and closing device 300, the sample rack 500 and the consumable rack 600 in the exemplary embodiment of the present disclosure may all be mounted in the nucleic acid purification region 110, the first taking and placing device 200 in the exemplary embodiment of the present disclosure may be moved or operated to the nucleic acid purification module 180, the sample rack 500, the consumable rack 600, the first lid opening and closing device 300 and the centrifugal transfer device 800, and take and place detection objects, such as the sample tube 900, or the like, and the detection objects include the sample tube 900, the PCR tube 1000, various pipette tips, or the like. The first lid opening and closing device 300 in the exemplary embodiment of the present disclosure may be configured to open or close a tube lid of the sample tube 900, the sample rack 500 in the exemplary embodiment of the present disclosure is configured to store the sample tube 900, and the consumable rack 600 in the exemplary embodiment of the present disclosure is configured to store the PCR tube 1000, reagents, various pipette tips, or the like.

The PCR detection device 700 and the second taking and placing device in the exemplary embodiment of the present disclosure may be both mounted in the PCR detection region 120, and the second taking and placing device in the exemplary embodiment of the present disclosure may be moved to the centrifugal transfer device 800 and the PCR detection device 700 and take and place the PCR tube, and has a similar function and structure to the first taking and placing device 200 in the exemplary embodiment of the present disclosure.

The centrifugal transfer device 800 in the exemplary embodiment of the present disclosure may have one part located in the nucleic acid purification region 110 and the other part located in the PCR detection region 120, the centrifugal transfer device 800 may be configured to perform centrifugal treatment on the PCR tube 1000, and using position characteristics and structural characteristics of the centrifugal transfer device 800, the centrifugal transfer device 800 may rotate to drive the PCR tube 1000 thereon to move from the nucleic acid purification region 110 to the PCR detection region 120, thereby simplifying a structure and integrating centrifugation and transfer functions.

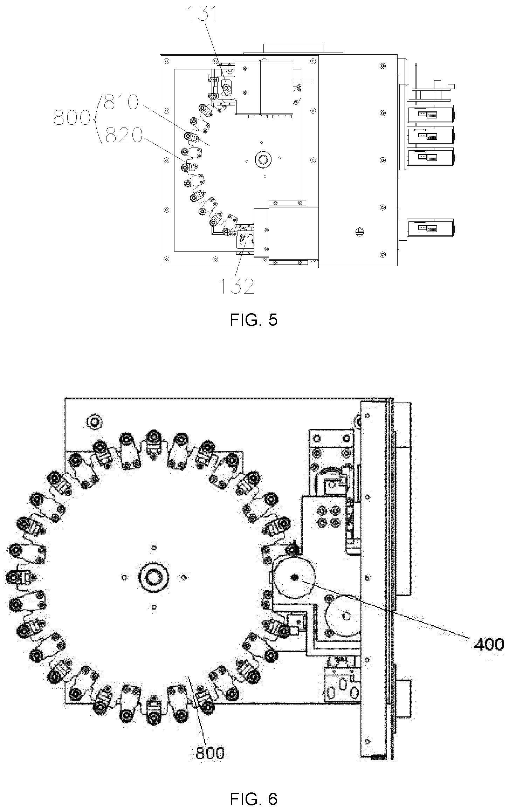

The structure of the centrifugal transfer device 800 according to the exemplary embodiment of the present disclosure will be described below with reference to FIGS. 5 to 18. The centrifugal transfer device 800 in the exemplary embodiment of the present disclosure may structurally include a rotary disc 810 and a plurality of mounting assemblies 820 hinged near an edge of the rotary disc 810, the mounting assembly 820 may be configured to fix and contain the PCR tube 1000, and during centrifugal rotation of the rotary disc 810, since the mounting assembly 820 may further rotate centrifugally under a centrifugal force, an efficiency and effect of centrifuging the PCR tube 1000 can be improved. In some embodiments, the rotary disc 810 may be a rotary circular disc.

As shown in FIGS. 5 to 9, in some embodiments, the centrifugal transfer device 800 may include: a rotary disc 810, a second lid opening and closing device 400 and a rotary driving member 3. The rotary disc 810 may be configured to mount the PCR tube 1000, the second lid opening and closing device 400 may have a grabbing portion 7, and the grabbing portion 7 can drive the tube lid on the PCR tube 1000 to move; the rotary driving member 3 may be in transmission connection with the rotary disc 810, and the rotary driving member 3 may have a rotary mode and a centrifugal mode; when in the rotary mode, the rotary driving member 3 may drive the PCR tube 1000 to move to a position below the grabbing portion 7; and when in the centrifugal mode, the rotary driving member 3 may drive the PCR tube 1000 to perform centrifugal motion.

In some embodiments, the PCR tube 1000 may be placed on the rotary disc 810, and a number of the PCR tubes 1000 may be one or more.

An origin trigger may be provided on the rotary disc 810, an origin position signal sensor may be provided on the rotary driving member 3, the rotary driving member 3 drives the rotary disc 810 to rotate, and when the origin trigger is rotated to a position of the origin position signal sensor, the origin position signal sensor senses the origin trigger, the rotary disc 810 stops rotating, and this position serves as a position origin; each PCR tube 1000 on the rotary disc 810 is rotated to a tube placing position of a moving mechanical arm with this position as a reference and stops, and in conjunction with hole positions on the rotary disc 810, a clamping jaw on the moving mechanical arm automatically loads all the hole positions with the PCR tubes 1000 or places a required number of PCR tubes 1000 on the hole positions of the rotary disc 810, and a pipetting device on the moving mechanical arm adds the required reagent or sample into the PCR tube 1000 with the lid opened, for example, adds a PCR reagent and a DNA or RNA template into the PCR tube 1000 with the lid opened.

When the rotary driving member 3 is switched to the rotary mode, the rotary disc 810 is driven to rotate, such that the PCR tube 1000 is moved to a position below the grabbing portion 7, thus facilitating the grabbing portion 7 to open or close the tube lid of the PCR tube 1000; when the rotary driving member 3 is switched to the centrifugal mode, the rotary disc 810 is driven to rotate at a high speed, and the PCR tube 1000 performs centrifugal motion, thus facilitating a centrifugal operation of the reagent.

The centrifugal transfer device 800 according to the exemplary embodiment of the present disclosure may include: the rotary disc 810, the second lid opening and closing device 400 and the rotary driving member 3; the rotary disc 810 may be configured to mount the PCR tube 1000, the second lid opening and closing device 400 may have the grabbing portion 7, and the grabbing portion 7 can drive the tube lid on the PCR tube 1000 to move; the rotary driving member 3 is in transmission connection with the rotary disc 810, and the rotary driving member 3 may have the rotary mode and the centrifugal mode; when in the rotary mode, the rotary driving member 3 may drive the PCR tube 1000 to move to the position below the grabbing portion 7; when in the centrifugal mode, the rotary driving member 3 may drive the PCR tube 1000 to perform centrifugal motion. The PCR tube 1000 is placed on the rotary disc 810, the rotary mode of the rotary driving member 3 is started, the PCR tube 1000 is moved to the position below the grabbing portion 7, and the tube lid on the PCR tube 1000 is driven to move by the grabbing portion 7 of the second lid opening and closing device 400, so as to complete a lid opening or closing operation; the centrifugal mode of the rotary driving member 3 is started, and the rotary disc 810 rotates at a high speed to drive the PCR tube 1000 to perform centrifugal motion, thus facilitating the centrifugal operation of the reagents in the PCR tube 1000; and lid opening and closing functions can be achieved, the reagents may be centrifuged, more functions are achieved, and the technical problem that in a prior art, an existing PCR tube 1000 lid opening and closing device can only achieve single automatic lid opening and closing functions is solved.

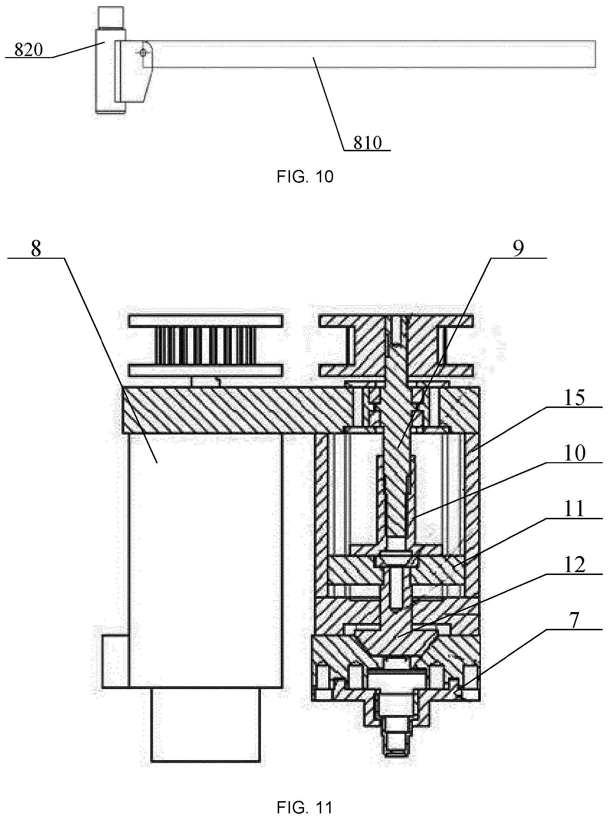

Based on the exemplary embodiment described above, as shown in FIG. 10, in an optional embodiment, the centrifugal transfer device 800 according to the present disclosure includes a mounting assembly 820, and the mounting assembly 820 may be a hanging basket. The PCR tube 1000 is placed in the hanging basket; a plurality of hanging baskets are provided, the plural hanging baskets are uniformly arranged in an outer circumference direction of the rotary disc 810, and the hanging baskets are hinged to the rotary disc 810; a tube lid placing portion 6 is provided between any two adjacent hanging baskets.

In some embodiments, sample tube placing holes in the rotary disc 810 are uniformly distributed in the rotary disc 810, the rotary disc 810 may be fixedly connected with the rotary driving member 3 by a connecting piece, the hanging baskets may be uniformly distributed and mounted on the rotary disc 810, the hanging baskets may swing flexibly, the tube lid placing portion 6 may be provided between any two hanging baskets, the tube lid placing portions 6 and the hanging baskets are alternately and uniformly distributed and mounted on the rotary disc 810, and the unscrewed tube lid is placed on the tube lid placing portion; in addition, the tube lid placing portion 6 may not be provided, if there exists only one PCR tube 1000, the tube lid may be always grabbed by the grabbing portion 7 without placement of the tube lid, and whether the tube lid placing portion 6 is provided is selected according to actual conditions; and the rotary driving member 3 moves to position each hole position with a reference that the origin trigger triggers the origin position signal sensor, and meanwhile, the rotary driving member 3 also achieves a high-speed rotary centrifugation function to serve as a centrifugal motor.

The PCR tube 1000 may be placed in the hanging basket, and the hanging basket may be connected with the rotary disc 810 by a rotating shaft; when the rotary disc 810 does not rotate, the hanging basket is kept perpendicular to a surface of the rotary disc 810 under the action of gravity, and at this point, the sample, the PCR tube 1000 and the tube lid may be taken and placed conveniently; during centrifugation, an included angle between the hanging basket and the rotary disc 810 becomes smaller under the action of a centrifugal force, such that the reagent or sample in the PCR tube 1000 may be conveniently centrifuged to a bottom of the tube.

As shown in FIG. 11, in an optional embodiment, the second lid opening and closing device 400 may include a gripper driving member 8, a connecting screw 9, a nut sleeve 10, a moving seat 11 and a wedge 12; the gripper driving member 8 may be in transmission connection with the connecting screw 9, the nut sleeve 10 may be provided on the connecting screw 9, the nut sleeve 10 may be connected with the wedge 12 through the moving seat 11, and the gripper driving member 8 may be configured to drive the connecting screw 9 to rotate in an axis direction of the connecting screw, so as to move the nut sleeve 10, the moving seat 11 and the wedge 12 in the axis direction of the connecting screw 9; the wedge 12 may have an inclined surface, the grabbing portion 7 may abut against the inclined surface, and the wedge 12 moves to drive the grabbing portion 7 to clamp or release the tube lid.

In some embodiments, the gripper driving member 8 may be a driving motor, a first belt pulley may be provided on an output shaft of the driving motor, a second belt pulley may be provided at an end of the connecting screw 9, and transmission of the first belt pulley and the second belt pulley may be implemented by a belt; the nut sleeve 10 may be in threaded connection with the connecting screw 9, the gripper driving member 8 drives the connecting screw 9 to rotate, the moving seat 11 and the wedge 12 connected with the nut sleeve 10 linearly move along the connecting screw 9, the nut sleeve 10 linearly moves back and forth by forward and reverse rotation of the connecting screw 9, and the wedge 12 is fixedly connected with the moving seat 11 by a connecting piece, such that the wedge 12 linearly moves back and forth; the inclined surface is provided on the wedge 12, the grabbing portion 7 specifically includes two fingers, corresponding inclined surfaces are provided on the fingers, and the inclined surface of the wedge 12 is matched with the corresponding inclined surfaces of the two fingers, thereby driving the two fingers to move close to and away from each other through the inclined surface of the wedge 12, so as to achieve functions of clamping and releasing the tube lid by tube lid grippers provided on the fingers.

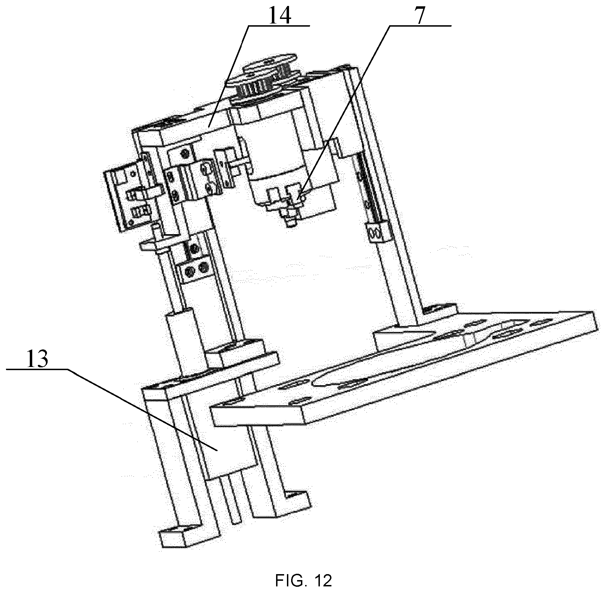

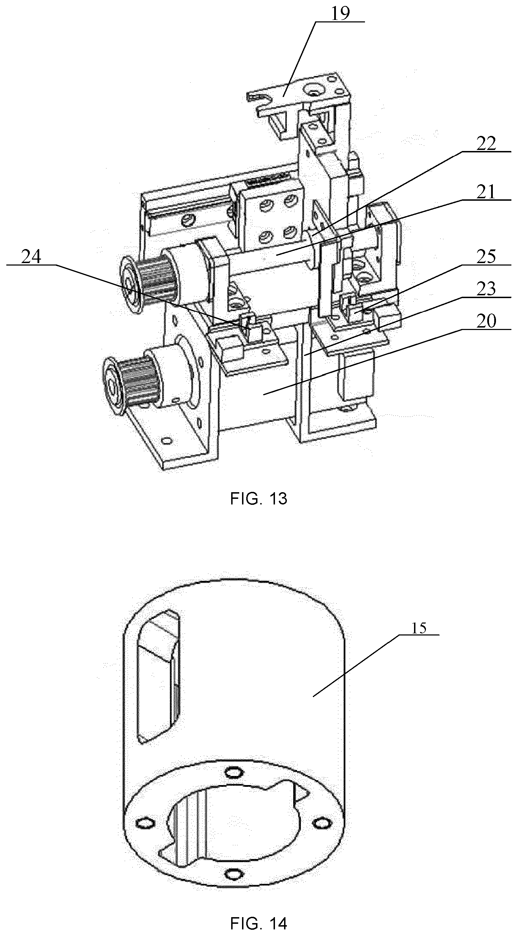

As shown in FIGS. 12 and 14, in an optional embodiment, the second lid opening and closing device 400 may further include a lifting driving member 13, a fixing mounting plate 14 and a fixing seat 15; the fixing seat 15 may be connected with the fixing mounting plate 14, a sliding groove may be provided in the fixing seat 15, the connecting screw 9 may penetrate through the fixing seat 15 into the sliding groove, and the nut sleeve 10 may be located in the sliding groove; the lifting driving member 13 may be connected with the fixing mounting plate 14, and the lifting driving member 13 may be configured to drive the fixing mounting plate 14 to move, so as to move the grabbing portion 7 towards or away from the PCR tube 1000.

In some embodiments, the lifting driving member 13 may be mounted on a lifting mounting frame, a sliding rail is provided on the lifting mounting frame, the fixing mounting plate 14 may be slidably connected with the sliding rail, the origin position signal sensor may be provided on the lifting mounting frame, a signal trigger may be provided on the fixing mounting plate 14, and the lifting driving member 13 moves up and down to drive the grabbing portion 7 to move up and down, so as to separate and combine the tube lid and a tube body; the lifting driving member 13 moves with a reference that the signal trigger triggers the origin position signal sensor.

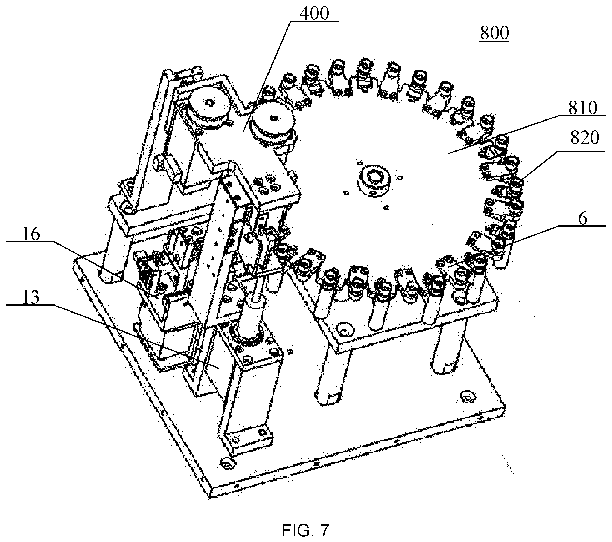

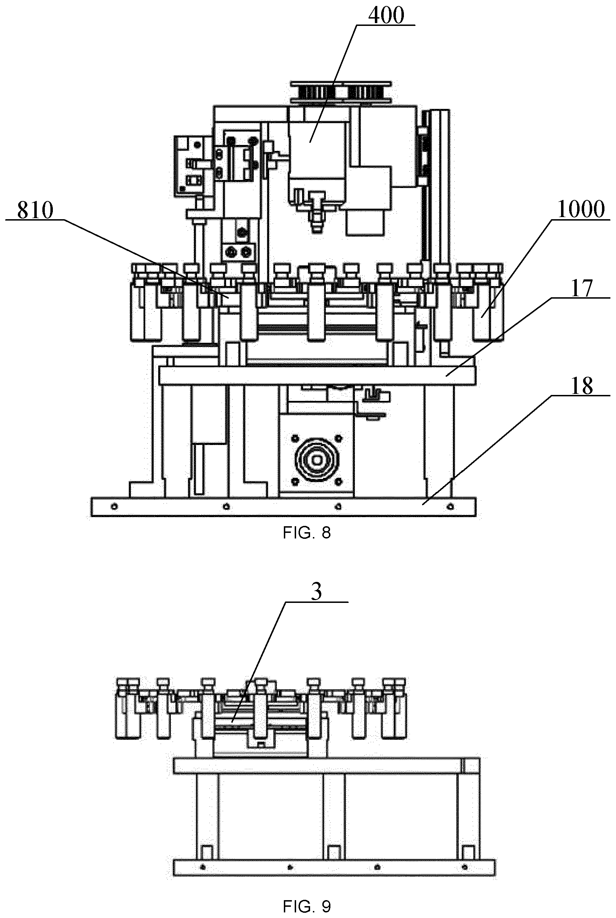

In an optional embodiment, the centrifugal transfer device 800 may further include a tube body fixing assembly 16 (shown in FIG. 7); a motor base plate 17 may be provided at a bottom of the rotary driving member 3, a fixing base plate 18 may be provided on the side of the motor base plate 17 away from the rotary driving member 3, and the second lid opening and closing device 400 and the tube body fixing assembly 16 may be both provided on the fixing base plate 18, as shown in FIG. 8; the tube body fixing assembly 16 may have a tube body clamp 19 (shown in FIG. 13) for clamping the PCR tube 1000.

In some embodiments, the fixing base plate 18 and the motor base plate 17 may be connected by a support, such that the fixing base plate 18 supports the motor base plate 17, the tube body fixing assembly 16 may be provided beside the rotary disc 810, and the tube body clamp 19 may clamp the PCR tube 1000 when the lid is opened, thereby avoiding that the PCR tube 1000 is inseparable from the tube lid due to too tight connection therebetween.

In an optional embodiment, as shown in FIG. 13, the tube body fixing assembly 16 may include a clamping driving member 20, a clamping screw 21 and a clamping nut 22; the clamping driving member 20 may be in transmission connection with the clamping screw 21, the clamping nut 22 is provided on the clamping screw 21, the clamping nut 22 is connected with the tube body clamp 19, and the clamping driving member 20 may be configured to drive the clamping screw 21 to rotate in an axis direction of the clamping screw, so as to move the tube body clamp 19 towards or away from the PCR tube 1000.

In an optional embodiment, a belt pulley may be provided at a driving end of the clamping driving member 20, and a belt pulley is also provided at an end of the clamping screw 21, such that the clamping driving member 20 drives the clamping screw 21 to rotate by cooperation of the two belt pulleys, so as to further drive the clamping nut 22 and the tube body clamp 19 connected with the clamping nut 22 to move back and forth, thereby fixing and releasing the tube body.

In an optional embodiment, the tube body fixing assembly 16 may further include a clamping mounting frame 23; the clamping driving member 20 may be connected with the clamping mounting frame 23, and the clamping mounting frame 23 may be provided with a limiting signal sensor 24 and an origin signal sensor 25.

In an optional embodiment, a guide rail may be provided on the clamping mounting frame 23, the tube body clamp 19 may move along the guide rail, a trigger may be provided on the tube body clamp 19, and the tube body clamp 19 moves with the trigger and trigger positions of the limiting signal sensor 24 and the origin signal sensor 25 as references, such that the tube body clamp 19 can only move between the limiting signal sensor 24 and the origin signal sensor 25.

In some embodiments, a lid opening working process is as follows: the PCR tube 1000 is placed in the mounting assembly 820, such as the hanging basket, on the rotary disc 810, the rotary mode of the rotary driving member 3 is started, the rotary disc 810 rotates to move the PCR tube 1000 to the position below the grabbing portion 7, the lifting driving member 13 drives the grabbing portion 7 to move towards the PCR tube 1000, the clamping driving member 20 drives the tube body clamp 19 to move towards the PCR tube 1000, and the tube body of the PCR tube 1000 is clamped by the tube body clamp 19; the gripper driving member 8 drives the connecting screw 9 to rotate, so as to open the two grippers of the grabbing portion 7, and after the two grippers move to the position of the tube lid, the gripper driving member 8 drives the connecting screw 9 to rotate reversely, the two grippers of the grabbing portion 7 get close to each other to clamp the tube lid, and the lifting driving member 13 drives the grabbing portion 7 to move away from the PCR tube 1000 to separate the tube lid from the tube body, thus completing the lid opening operation.

A specific lid closing working process is as follows: after the grabbing portion 7 grabs the tube lid, the lifting driving member 13 drives the grabbing portion 7 to move towards the tube body, the tube lid extends to the tube body, the gripper driving member 8 drives the connecting screw 9 to rotate, and the two grippers of the grabbing portion 7 get away from each other to release the tube lid, thus completing the lid closing operation.

In the embodiment of the present disclosure, the lids may be opened and closed continuously or individually.

When centrifugation is required, the centrifugal mode of the rotary driving member 3 is started, the rotary disc 810 rotates at a high speed to swing the hanging basket, and the reagent in the tube body of the PCR tube 1000 performs centrifugal motion to finish the centrifugal operation.

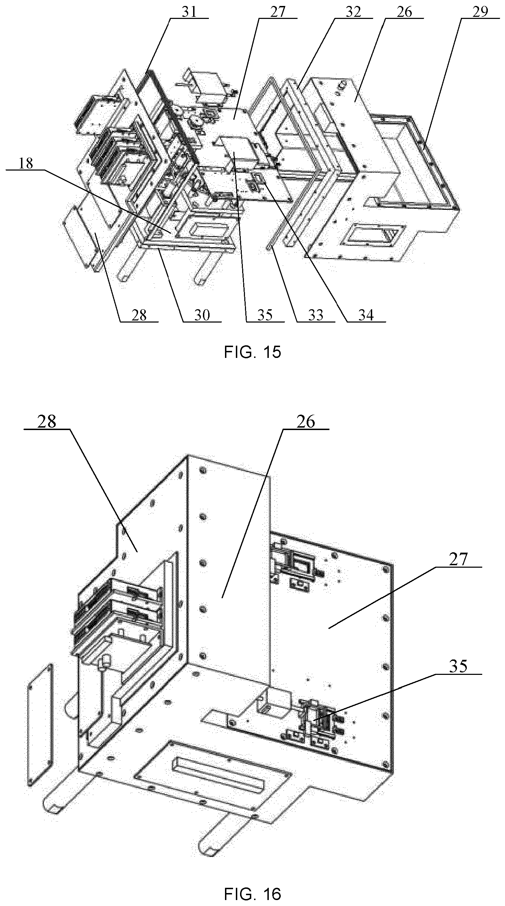

The reagent or sample in the test tube may contaminate an environment in the process of opening and closing the lid, and in order to avoid this situation, on the basis of the above embodiment, as shown in FIGS. 15 and 16, in an optional embodiment, the centrifugal transfer device 800 may further include an outer housing 26, an upper cover plate 27 and a rear cover plate 28; the upper cover plate 27 and the rear cover plate 28 may be both connected with the outer housing 26, the fixing base plate 18 may be connected with the outer housing 26 and the rear cover plate 28, the fixing base plate 18, the upper cover plate 27, the rear cover plate 28 and the outer housing 26 enclose an accommodating cavity, and the second lid opening and closing device 400 may be located in the accommodating cavity.

In some embodiments, the accommodating cavity enclosed by the fixing base plate 18, the upper cover plate 27, the rear cover plate 28 and the outer housing 26 packages the second lid opening and closing device 400, the rotary disc 810, the rotary driving member 3 and the tube body fixing assembly 16 and isolates them from the outside.

In an optional embodiment, as shown in FIG. 15, an upper cover sealing strip 29 may be provided between the outer housing 26 and the upper cover plate 27; the fixing base plate 18 may be provided with a lower sealing strip 30; a first sealing strip 31 may be provided between the rear cover plate 28 and the outer housing 26; an inner frame 32 may be provided in the accommodating cavity, and a second sealing strip 33 is provided between the inner frame 32 and the outer housing 26.

In an optional embodiment, the lower sealing strip 30 may be mounted on the fixing base plate 18, and the outer housing 26 may be connected with the fixing base plate 18, such that the outer housing 26 tightly presses the lower sealing strip 30 to seal a lower portion; the upper cover sealing strip 29 may be mounted on the outer housing 26, and the upper cover plate 27 may be connected with the outer housing 26, such that the upper cover sealing strip 29 is pressed tightly to seal an upper portion; the inner frame 32 may be connected with the outer housing 26, such that the second sealing strip 33 is pressed tightly to seal a side surface; the rear cover plate 28 may be connected with the outer housing 26 and the fixing base plate 18, such that the first sealing strip 31 and the lower sealing strip 30 are pressed tightly to seal a rear surface.

As shown in FIGS. 16, 17 and 18, in an optional embodiment, the centrifugal transfer device 800 may further include a door opening and closing assembly 35; the upper cover plate 27 may be provided with an opening, the door opening and closing assembly 35 may be provided with a door pressing plate 36, the opening may be plugged by the door pressing plate 36, and a door-plate sealing strip 34 is provided between the door opening and closing assembly 35 and the upper cover plate 27.

In some embodiments, a plurality of door opening and closing assemblies 35 may be provided, the door opening and closing assemblies 35 are provided in one-to-one correspondence to the openings and the door-plate sealing strips 34, and the door-plate sealing strips 34 may be mounted on the upper cover plate 27.

In an optional embodiment, the door opening and closing assembly 35 may include a linear motor 37, a door frame 38, a first spring hanger 39, a second spring hanger 40 and an elastic member 41; the linear motor 37 may be provided on the upper cover plate 27, the linear motor 37 may be in transmission connection with the door frame 38, the first spring hanger 39 may be provided on the door frame 38, a kidney shape hole 42 may be provided in the door frame 38, and the door pressing plate 36 may extend into the kidney shape hole 42 through the second spring hanger 40; one end of the elastic member 41 may be connected with the first spring hanger 39, the other end of the elastic member 41 may be connected with the second spring hanger 40, and the elastic member 41 may be configured to give the door pressing plate 36 a tendency to move towards the door frame 38.

In some embodiments, as shown in FIGS. 15 to 17, a first door enclosure and a second door enclosure may be provided on the upper cover plate 27, a motor mounting base is mounted on the upper cover plate 27, the linear motor 37 is mounted on the motor mounting base, a guide rail assembly is mounted on the upper cover plate 27, the door frame 38 is mounted on a slider of the guide rail assembly through a support, the first spring hanger 39 is mounted on the door frame 38, the door pressing plate 36 is connected with the second spring hanger 40, shafts at two ends of the second spring hanger 40 may be inserted into the kidney shape holes 42 of the door frame 38, hooks at two ends of the elastic member 41 are hooked on the first spring hanger 39 and the second spring hanger 40 to hang the door pressing plate 36 in the kidney shape hole 42 for movement, a screw of the linear motor 37 is connected with the door frame 38, a trigger sensing device is connected with the door frame 38, a first sensor and a second sensor may be fixed on the upper cover plate 27, and the second door enclosure may be fixed on the support and the slider of the guide rail assembly.

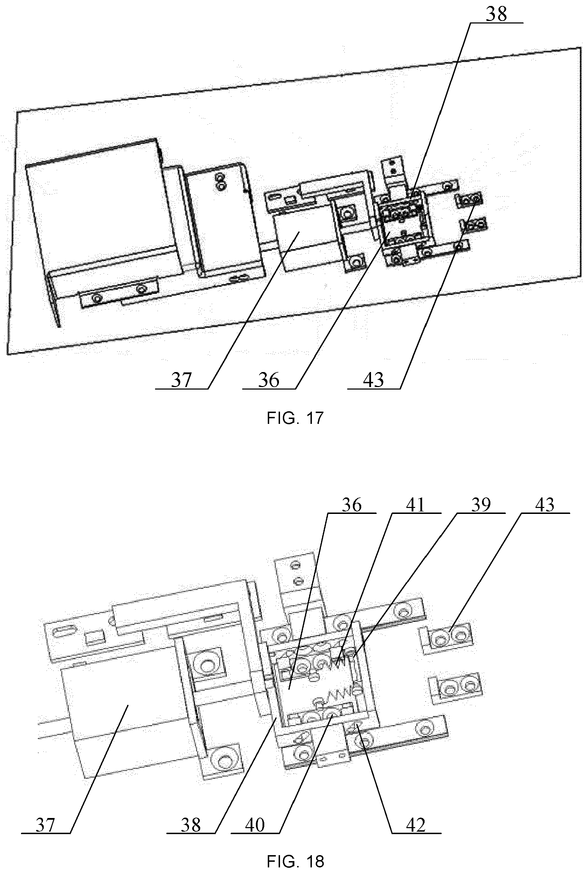

As shown in FIG. 18, when the door frame 38 is moved to a limiting position and the linear motor 37 moves forwards, the door pressing plate 36 moves downwards to tightly press the door-plate sealing strip 34, such that the opening of the upper cover plate 27 is sealed, thereby preventing the internal sample or reagent from contaminating the outside.

In an optional embodiment, the upper cover plate 27 may be provided with a limiting block 43; the linear motor 37 drives the door frame 38 to move towards the limiting block 43, such that the door pressing plate 36 abuts against the limiting block 43, and the door pressing plate 36 moves along the kidney shape hole 42 to tightly press the door-plate sealing strip 34.

In an optional embodiment, as shown in FIG. 18, the linear motor 37 is activated, and the screw of the linear motor 37 moves linearly forwards, such that the door frame 38 slides on the guide rail assembly; when the door pressing plate 36 contacts the limiting block 43 and the linear motor 37 moves forwards, an assembly of the door pressing plate 36 and the second spring hanger 40 may slide in the door frame 38 along the kidney shape hole 42, and move downwards in a vertical direction, so as to press a door sealing strip downwards, thereby achieving a door sealing purpose; when the linear motor 37 moves backwards, the door frame 38 moves backwards along the guide rail assembly, and at this point, the assembly of the door pressing plate 36 and the second spring hanger 40 moves upward under a pulling force of the elastic member, such that the door pressing plate 36 is separated from the door-plate sealing strip 34, the opening of the upper cover plate 27 is opened, and meanwhile, the second door enclosure always moves with the door frame 38.

The arrangement of the sealing strips between the outer housing and the cover plates of the centrifugal transfer device 800 may achieve an integral sealing effect of the centrifugal transfer device 800, such that the internal reagent or sample may be prevented from polluting the outside. Optionally, by the arrangement of a door assembly, a door sealing effect may be further achieved, such that the internal reagent or sample may be further prevented from polluting the outside.

A specific method to which the full-automatic gene analysis apparatus according to the exemplary embodiment of the present disclosure is applied may include specific steps of nucleic acid extraction, PCR system construction, centrifugal treatment, PCR detection, or the like; the nucleic acid extraction step in the exemplary embodiment of the present disclosure may include: the first taking and placing device 200 (shown in FIG. 19) transfers the sample tube 900 on the sample rack 500 to the first lid opening and closing device 300, so as to open a sample tube lid by the first lid opening and closing device, and then, the first taking and placing device clamps a first pipette tip on the consumable rack 600, allows the first pipette tip to suck a sample in the sample tube 900, and places the sample sucked by the first pipette tip in the nucleic acid purification module 180 for nucleic acid extraction.

The PCR system construction step S2 in the exemplary embodiment of the present disclosure may include: the first taking and placing device 200 transfers the PCR tube 1000 on the consumable rack 600 to the centrifugal transfer device 800, the PCR tube lid is opened by the second lid opening and closing device 400, and the first taking and placing device 200 then takes a second pipette tip on the consumable rack 600, allows the second pipette tip to suck a reagent, adds the reagent into the PCR tube 1000, then takes a third pipette tip on the consumable rack 600, allows the third pipette tip to suck a gene sample extracted in the nucleic acid purification module 180, and adds the gene sample into the PCR tube 1000.

The centrifugal treatment step in the exemplary embodiment of the present disclosure may include: the PCR tube lid is closed by the second lid opening and closing device 400, and then, the centrifugal transfer device 800 performs rotary centrifugal treatment on the PCR tube 1000 and transfers the PCR tube 1000 after the centrifugal treatment to the PCR detection region 120.

The PCR detection step in the exemplary embodiment of the present disclosure may include: the second taking and placing device transfers the PCR tube 1000 located in the PCR detection region 120 to the PCR detection device 700 for detection.

Specifically, in the above-mentioned steps, before the full-automatic gene analysis apparatus works, the sample tube 900 filled with the sample is required to be placed on the sample rack 500; in specific work, the first taking and placing device 200 first grabs the sample tube 900 on the sample rack 500 to the first lid opening and closing device 300 for the lid opening operation; after the lid of the sample tube 900 is opened, the first taking and placing device 200 in the exemplary embodiment of the present disclosure is moved to a pipette tip region of the consumable rack 600 to take the first pipette tip, is then moved to the sample tube 900 with the lid opened by the first lid opening and closing device 300 again, allows the first pipette tip to suck the sample in the sample tube 900, is then moved to a position above a middle part of the reagent strip of the nucleic acid purification module 180, drops the sample sucked in the first pipette tip into the reagent strip, and then discards the first pipette tip to the waste tip collecting region. After the above-mentioned step is completed, the first lid opening and closing device 300 again closes the sample tube lid, and then, the first taking and placing device 200 places the sample tube 900 with the sample tube lid closed by the first lid opening and closing device 300 back again into the sample rack 500.

While the reagent strip of the above-mentioned nucleic acid purification module 180 performs nucleic acid extraction, the first taking and placing device 200 in the exemplary embodiment of the present disclosure grabs the PCR tube 1000 in a PCR tube region in the consumable rack 600 to the centrifugal transfer device 800, the PCR tube lid is opened by the second lid opening and closing device 400, and then, the first taking and placing device 200 is moved to the consumable rack 600 again to take the second pipette tip, adds the reagent into the PCR tube 1000 with the lid opened on the centrifugal transfer device after the reagent is sucked in a reagent region of the consumable rack 600, and then discards the second pipette tip.

After the nucleic acid purification module 180 in the exemplary embodiment of the present disclosure completes the nucleic acid extraction, the first taking and placing device 200 may be moved to the consumable rack 600 again to take the third pipette tip, and is then moved to the nucleic acid purification module 180, an appropriate quantity of extracted DNA or RNA templates are sucked and added into the reagent-added PCR tube 1000 with the lid opened on the centrifugal transfer device 800, and then, the third pipette tip is discarded; or the first taking and placing device 200 is directly moved to the nucleic acid purification module 180 after taking the third pipette tip, all the DNA or RNA templates extracted by the nucleic acid purification module 180 are sucked, an appropriate quantity of DNA or RNA templates are added into the reagent-added PCR tube 1000 with the lid opened, and the remaining DNA or RNA templates are transferred to a template buffer tube of the template buffer region for later use; then, the lid of the PCR tube is closed by the second lid opening and closing device 400.

After the above-mentioned step is completed, a centrifugal transfer module performs rotary centrifugation, and the reagent, and the DNA or RNA template in the PCR tube 1000 are centrifuged to the bottom of the test tube; after the centrifugation by the PCR tube 1000 is completed, the centrifugal transfer device 800 in the exemplary embodiment of the present disclosure is rotated by a certain angle, and the PCR tube 1000 thereon is transferred to the PCR detection region 120.

Finally, the second taking and placing device in the PCR detection region 120 may move, to the PCR detection device 700, the PCR tube 1000 on the centrifugal transfer device 800 transferred into the PCR detection region 120, the PCR detection device 700 includes a plurality of PCR detection mechanisms, and the plurality of PCR detection mechanisms are sequentially arranged along a preset direction; the second taking and placing device sequentially places the PCR tubes 1000 into the plurality of PCR detection mechanisms, detection is performed after all the hole positions of the PCR detection mechanism are loaded with the PCR tubes 1000, and meanwhile, the second taking and placing device in the exemplary embodiment of the present disclosure continues to perform taking and placing operations between the centrifugal transfer device 800 and the PCR detection device 700, and both operations are performed simultaneously, thereby improving a working efficiency; after one PCR detection mechanism completes detection, the second taking and placing device discards the detected PCR tube 1000 to a PCR waste bin (not shown).

Based on the above structure, in order to prevent aerosol in the PCR detection region 120 from entering the nucleic acid purification region 110 from the PCR buffer region 130, in the exemplary embodiment of the present disclosure, the PCR buffer region 130 therebetween is subjected to sealing treatment, the PCR buffer region 130 is designed as a sealed chamber, the second lid opening and closing device 400 and the centrifugal transfer device 800 are both located in the sealed chamber, and the sealed chamber is provided with a first opening 131 located in the nucleic acid purification region 110 and a second opening 132 located in the PCR detection region 120; the first opening 131 is provided with a first door (not shown) for opening and closing the first opening, and the second opening 132 is provided with a second door (not shown) for opening and closing the second opening; under control of a control system of the apparatus, the second door in the exemplary embodiment of the present disclosure is closed when the first door in the exemplary embodiment of the present disclosure is opened, and the second door in the exemplary embodiment of the present disclosure is opened when the first door in the exemplary embodiment of the present disclosure is closed, thus ensuring that the nucleic acid purification region 110 and the PCR detection region 120 are sealed with respect to each other. Correspondingly, in order to transfer the PCR tube 1000 and open the lid thereof, the centrifugal transfer device 800 and the second lid opening and closing device 400 in the exemplary embodiment of the present disclosure are both mounted in the sealed chamber, the second lid opening and closing device 400 is configured to perform the lid opening and closing operation on the PCR tube 1000 on the centrifugal transfer device 800, and the rotary disc 810 of the centrifugal transfer device 800 in the exemplary embodiment of the present disclosure is located below the first opening 131 and the second opening 132, such that the PCR tube 1000 thereon can be moved to positions below the first opening 131 and the second opening 132 under rotation, thus facilitating the taking and placing operations of the first taking and placing device 200 and the second taking and placing device.

In addition, in order to further improve a sealing effect and prevent the aerosol in the PCR detection region 120 from entering the nucleic acid purification region 110, a mounting chamber 160 may be provided at the lower portion of the body 100 in the exemplary embodiment of the present disclosure, and a negative pressure suction device 1100 and an air treatment device 1200 may be mounted in the mounting chamber 160; the negative pressure suction device 1100 may be respectively communicated with the PCR detection region 120 and the air treatment device 1200, and the air treatment device 1200 is communicated with outside atmosphere; in the exemplary embodiment of the present disclosure, a one-way valve (not shown) is further provided between the sealed chamber and the PCR detection region 120, such that a gas stream can only unidirectionally flow to the PCR detection region 120 from the sealed chamber. When the apparatus runs normally, the negative pressure suction device 1100 always operates to draw out gas in the PCR detection region 120, and due to the action of the one-way valve, part of gas in the PCR buffer region 130 flows into the PCR detection region 120, such that a certain pressure difference is kept between the PCR buffer region 130 and the PCR detection region 120, and a gas pressure in the nucleic acid purification region 110 is greater than a gas pressure in the PCR buffer region 130 which is greater than a gas pressure in the PCR detection region 120. Since the gas pressure in the nucleic acid purification region 110 is greater than the gas pressure in the PCR buffer region 130, when the first door 131 is opened, the gas flows from the nucleic acid purification region 110 into the PCR buffer region 130 and cannot flow reversely, and when the second door 132 is opened, the gas in the PCR buffer region 130 flows into the PCR detection region 120 and cannot flow reversely, such that the aerosol is prevented from diffusing from the PCR detection region 120 to the nucleic acid purification region 110, thus avoiding pollution to the nucleic acid purification region 110.

With reference to FIGS. 19 and 20, the first taking and placing device 200 in the exemplary embodiment of the present disclosure may include a motion mechanism 210 and a first clamping mechanism 220 connected to each other, and the motion mechanism 210 may be configured to drive the first clamping mechanism 220 to translate and lift and drop; in order to enable the motion mechanism 210 in the exemplary embodiment of the present disclosure to move to a desired position, in the exemplary embodiment of the present disclosure, the motion mechanism is designed to be movable along three mutually perpendicular directions, and since such a moving structure is an existing structure, the exemplary embodiment of the present disclosure does not give too many descriptions of a moving manner of the first taking and placing device 200.

The first taking and placing device 200 in the exemplary embodiment of the present disclosure is mainly structurally improved in the first clamping mechanism 220; the first clamping mechanism 220 in the exemplary embodiment of the present disclosure includes two clamping arms 230, each clamping arm 230 is provided with a first clamping structure 231 and a second clamping structure 232, the two first clamping structures 231 are fitted to form a first clamping portion, and the two second clamping structures 232 are fitted to form a second clamping portion.

Specifically, the first clamping structure 231 may be an arc groove formed in an inner side of the clamping arm 230, the second clamping structure 232 may be a clamping block provided at a lower end of the clamping arm 230, a clamping ring can be formed by the two arc grooves to clamp the sample tube 900, and the PCR tube 1000 can be clamped by cooperation of the two clamping blocks, such that the first clamping mechanism 220 has double clamping functions.

In addition, in order to pick up the pipette tips, suck liquid by the pipette tips and drain the pipette tips, the first taking and placing device 200 in the exemplary embodiment of the present disclosure further includes the pipetting device (not shown), and the pipetting device in the exemplary embodiment of the present disclosure may perform pipetting operations, such as liquid suction, liquid drainage, or the like, after the pipette tips are taken; that is, the first taking and placing device 200 in the present disclosure may achieve the double clamping functions and also integrate the pipetting device to perform the pipetting operation, such that on the one hand, the device is multipurpose, and a structure of the apparatus is simplified, and on the other hand, full-automatic operation of the apparatus is guaranteed.

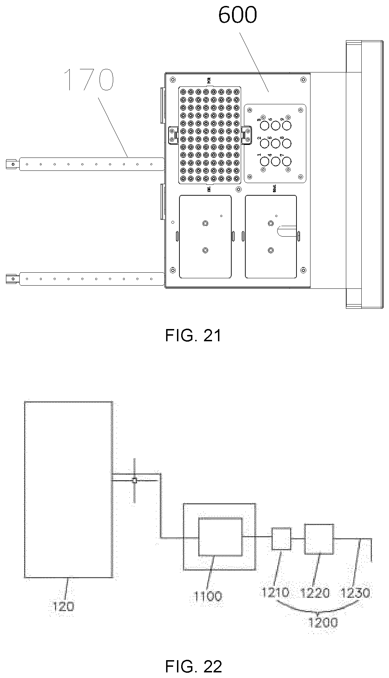

In addition, with reference to FIG. 21, a structure of the consumable rack 600 is further improved in the exemplary embodiment of the present disclosure, the body 100 may be provided with a guide rail 170, the consumable rack 600 in the exemplary embodiment of the present disclosure may be slidably mounted on the guide rail 170, the consumable rack 600 forms a drawer-type structure to facilitate replacement of consumables, and preferably, the consumable rack 600 in the exemplary embodiment of the present disclosure is provided with a refrigeration module, thus guaranteeing chemical stability of various reagents thereon.

With reference to FIG. 22, a structure of the air treatment device 1200 is further optimized in the exemplary embodiment of the present disclosure, the air treatment device 1200 may include an air filter 1210, a reagent solution filter 1220 and an exhaust pipe 1230, the negative pressure suction device 1100, the air filter 1210, the reagent solution filter 1220 and the exhaust pipe 1230 are communicated sequentially, and after the air in the PCR detection region 120 is sucked by the negative pressure suction device 1100, the air filter 1210 and the reagent solution filter 1220 in the exemplary embodiment of the present disclosure perform double filtration on the air, thus guaranteeing a filtering effect; the filtered gas is directly exhausted by the exhaust pipe 1230, thus avoiding air pollution.

In conclusion, with the full-automatic gene analysis apparatus and the application method of the apparatus according to the exemplary embodiment of the present disclosure, plural purposes are achieved with one apparatus while automatic detection is realized, and manual intervention is avoided in the whole process, thus improving the detection efficiency, guaranteeing detection accuracy, simplifying a required experimental apparatus, and reducing the space for detection.

### Industrial applicability

The present disclosure provides the full-automatic gene analysis apparatus and the gene analysis method; the full-automatic gene analysis apparatus achieves plural purposes while realizing automatic detection, and manual intervention is avoided in the whole process, thus improving the detection efficiency, guaranteeing the detection accuracy, simplifying the required experimental apparatus, and reducing the space for detection.

Furthermore, it may be understood that the full-automatic gene analysis apparatus, the PCR detection device, the nucleic acid purification module, the centrifugal transfer device, or the like, in the present disclosure are reproducible and may be applied to various industrial applications. For example, the full-automatic gene analysis apparatus according to the present disclosure may be applied to a gene analysis experiment in which automatic detection is required.

## Claims

1. A full-automatic gene analysis apparatus, **characterized by** comprising: a body (100), a first taking and placing device (200), a second taking and placing device, a first lid opening and closing device (300), a sample rack (500), a consumable rack (600), a PCR detection device (700), a centrifugal device (800) and a nucleic acid purification module (180), wherein
the body (100) comprises a nucleic acid purification region (110), a PCR detection region (120) and a PCR buffer region (130);
the first taking and placing device (200), the first lid opening and closing device (300), the sample rack (500), the consumable rack (600) and the nucleic acid purification module (180) are all mounted in the nucleic acid purification region (110), and the first taking and placing device (200) is configured to be moved, the sample rack (500), the consumable rack (600), the first lid opening and closing device (300) and the centrifugal device (800), and take and place a sample tube (900) or a PCR tube (1000) or perform a pipetting operation;
the PCR detection device (700) and the second taking and placing device are both mounted in the PCR detection region (120), and the second taking and placing device is configured to be moved to the PCR detection device (700) and take and place the PCR tube (1000); and
the centrifugal device (800) is mounted in the PCR buffer region (130); **characterized in that**
the full-automatic gene analysis apparatus further comprises a second lid opening and closing device that is mounted in the PCR buffer region (130);
the centrifugal device (800) is a centrifugal transfer device wherein one part of the centrifugal transfer device (800) is located in the nucleic acid purification region (110), the other part of the centrifugal transfer device is located in the PCR detection region (120), and the centrifugal transfer device (800) is configured to be rotated to drive the PCR tube (1000) on the centrifugal transfer device to move into the nucleic acid purification region (110) or the PCR detection region (120);
the first taking and placing device (200) is configured to be moved to the nucleic acid purification module (180); and
the second taking and placing device is configured to be moved to the centrifugal transfer device (800);
**and in that** the centrifugal transfer device (800) comprises a rotary disc (810) and a rotary driving member (3), wherein
the rotary disc (810) is configured to mount the PCR tube (1000), the second lid opening and closing device (400) has a grabbing portion (7), and the grabbing portion (7) is configured to drive a tube lid on the PCR tube (1000) to move; and
the rotary driving member (3) is in transmission connection with the rotary disc (810), and the rotary driving member (3) has a rotary mode and a centrifugal mode, wherein
when in the rotary mode, the rotary driving member (3) drives the PCR tube (1000) to move to a position below the grabbing portion (7); and
when in the centrifugal mode, the rotary driving member (3) drives the PCR tube (1000) to perform centrifugal motion,
the centrifugal transfer device (800) further comprises mounting assemblies (820), wherein
the PCR tubes (1000) are placed in the mounting assemblies (820), plural mounting assemblies (820) are provided, the plural mounting assemblies (820) are uniformly arranged along an outer circumference direction of the rotary disc (810), and the mounting assemblies (820) are hinged to the rotary disc (810); and
a tube lid placing portion (6) is provided between any two adjacent mounting assemblies (820);
the second lid opening and closing device (400) comprises a gripper driving member (8), a connecting screw (9), a nut sleeve (10), a moving seat (11) and a wedge (12), wherein
the gripper driving member (8) is in transmission connection with the connecting screw (9), the nut sleeve (10) is provided on the connecting screw (9), the nut sleeve (10) is connected with the wedge (12) through the moving seat (11), and the gripper driving member (8) is configured to drive the connecting screw (9) to rotate in an axis direction of the connecting screw, so as to move the nut sleeve (10), the moving seat (11) and the wedge (12) in the axis direction of the connecting screw (9); and
the wedge (12) has an inclined surface, the grabbing portion (7) abuts against the inclined surface, and the wedge (12) moves to drive the grabbing portion (7) to clamp or release the tube lid; **and in that**
the second lid opening and closing device (400) further comprises a lifting driving member (13), a fixing mounting plate (14) and a fixing seat (15), wherein
the fixing seat (15) is connected with the fixing mounting plate (14), a sliding groove is provided in the fixing seat (15), the connecting screw (9) penetrates through the fixing seat (15) into the sliding groove, and the nut sleeve (10) is located in the sliding groove; and
the lifting driving member (13) is connected with the fixing mounting plate (14), and the lifting driving member (13) is configured to drive the fixing mounting plate (14) to move, so as to move the grabbing portion (7) towards or away from the PCR tube (1000).

2. The full-automatic gene analysis apparatus according to claim 1, wherein the PCR buffer region (130) is provided across the nucleic acid purification region (110) and the PCR detection region (120), the PCR buffer region (130) is a sealed chamber, and the sealed chamber is provided with a first opening (131) located in the nucleic acid purification region (110) and a second opening (132) located in the PCR detection region (120), wherein
the first opening (131) is provided with a first door for opening and closing the first opening, and the second opening (132) is provided with a second door for opening and closing the second opening; and
the centrifugal transfer device (800) and the second lid opening and closing device (400) are both located within the sealed chamber and located below the first opening (131) and the second opening (132).

3. The full-automatic gene analysis apparatus according to claim 2, further comprising a negative pressure suction device (1100) and an air treatment device (1200), wherein the negative pressure suction device (1100) is respectively communicated with the PCR detection region (120) and the air treatment device (1200), the air treatment device (1200) is communicated with outside atmosphere, and the negative pressure suction device is configured to provide a negative pressure effect such that a gas pressure of the nucleic acid purification region is greater than that of the PCR buffer region, and a gas pressure of the PCR buffer region is greater than that of the PCR detection region.

4. The full-automatic gene analysis apparatus according to claim 3, wherein the air treatment device (1200) comprises an air filter (1210), a reagent solution filter (1220) and an exhaust pipe (1230), and the negative pressure suction device (1100), the air filter (1210), the reagent solution filter (1220) and the exhaust pipe (1230) are communicated sequentially.

5. The full-automatic gene analysis apparatus according to claim 2, wherein a one-way valve is provided between the sealed chamber and the PCR detection region (120) for allowing a unidirectional gas stream flow from the sealed chamber to the PCR detection region.

6. The full-automatic gene analysis apparatus according to any one of claims 1 to 5, wherein the first taking and placing device (200) comprises a motion mechanism (210) and a first clamping mechanism (220) connected to each other, and the motion mechanism (210) is configured to drive the first clamping mechanism (220) to translate and lift and drop; and
the first clamping mechanism (220) comprises two clamping arms (230), each clamping arm (230) is provided with a first clamping structure (231) and a second clamping structure (232), wherein two first clamping structures (231) are fitted to form a first clamping portion, and two second clamping structures (232) are fitted to form a second clamping portion,
the first clamping structure (231) is an arc groove formed in an inner side of the clamping arm (230), and/or the second clamping structure (232) is a clamping block provided at a lower end of the clamping arm (230);
the first taking and placing device (200) further comprises a pipetting device configured to perform a pipetting operation after a pipette tip is taken.

7. The full-automatic gene analysis apparatus according to any one of claims 1 to 5, wherein the PCR detection device (700) comprises a plurality of PCR detection mechanisms, the plurality of PCR detection mechanisms are sequentially arranged in a preset direction, the second taking and placing device is configured to sequentially place PCR tubes (1000) into the plurality of PCR detection mechanisms, the full-automatic gene analysis apparatus being configured for performing detection after all hole positions of the PCR detection mechanisms have been loaded with the PCR tubes.

8. The full-automatic gene analysis apparatus according to any one of claims 1 to 5, wherein
the centrifugal transfer device (800) further comprises a tube body fixing assembly (16);
a motor base plate (17) is provided at a bottom of the rotary driving member (3), a fixing base plate (18) is provided on a side of the motor base plate (17) away from the rotary driving member (3), and the second lid opening and closing device (400) and the tube body fixing assembly (16) are both provided on the fixing base plate (18); and
the tube body fixing assembly (16) has a tube body clamp (19) configured to clamp the PCR tube (1000),
the tube body fixing assembly (16) comprises a clamping driving member (20), a clamping screw (21) and a clamping nut (22),
wherein the clamping driving member (20) is in transmission connection with the clamping screw (21), the clamping nut (22) is provided on the clamping screw (21), the clamping nut (22) is connected with the tube body clamp (19), and the clamping driving member (20) is configured to drive the clamping screw (21) to rotate in an axis direction of the clamping screw, so as to move the tube body clamp (19) towards or away from the PCR tube (1000).

9. The full-automatic gene analysis apparatus according to claim 8, wherein
the tube body fixing assembly (16) further comprises a clamping mounting frame (23),
wherein the clamping driving member (20) is connected with the clamping mounting frame (23), and the clamping mounting frame (23) is provided with a limiting signal sensor (24) and an origin signal sensor (25).

10. The full-automatic gene analysis apparatus according to claim 8, wherein
the centrifugal transfer device (800) further comprises an outer housing (26), an upper cover plate (27) and a rear cover plate (28),
wherein the upper cover plate (27) and the rear cover plate (28) are both connected with the outer housing (26), the fixing base plate (18) is respectively connected with the outer housing (26) and the rear cover plate (28), the fixing base plate (18), the upper cover plate (27), the rear cover plate (28) and the outer housing (26) form an accommodating cavity, and the second lid opening and closing device (400) is located in the accommodating cavity,
an upper cover sealing strip (29) is provided between the outer housing (26) and the upper cover plate (27);
the fixing base plate (18) is provided with a lower sealing strip (30);
a first sealing strip (31) is provided between the rear cover plate (28) and the outer housing (26); and
an inner frame (32) is provided in the accommodating cavity, and a second sealing strip (33) is provided between the inner frame (32) and the outer housing (26);
the centrifugal transfer device (800) further comprises a door opening and closing assembly (35); and
the upper cover plate (27) is provided with an opening, the door opening and closing assembly (35) has a door pressing plate (36), the opening can be plugged by the door pressing plate (36), and a door-plate sealing strip (34) is provided between the door opening and closing assembly (35) and the upper cover plate (27).

11. The full-automatic gene analysis apparatus according to claim 10, wherein
the door opening and closing assembly (35) comprises a linear motor (37), a door frame (38), a first spring hanger (39), a second spring hanger (40) and an elastic member (41), wherein
the linear motor (37) is provided on the upper cover plate (27), the linear motor (37) is in transmission connection with the door frame (38), the first spring hanger (39) is provided on the door frame (38), a kidney shape hole (42) is provided in the door frame (38), and the door pressing plate (36) extends into the kidney shape hole (42) through the second spring hanger (40); and
one end of the elastic member (41) is connected with the first spring hanger (39), the other end of the elastic member (41) is connected with the second spring hanger (40), and the elastic member (41) is configured to give the door pressing plate (36) a tendency to move towards the door frame (38).

12. The full-automatic gene analysis apparatus according to claim 11, wherein
the upper cover plate (27) is provided with a limiting block (43); and
the linear motor (37) drives the door frame (38) to move towards the limiting block (43), such that the door pressing plate (36) abuts against the limiting block (43), and the door pressing plate (36) moves along the kidney shape hole (42) to tightly press the door-plate sealing strip (34).

13. A gene analysis method, to which the full-automatic gene analysis apparatus according to any one of claims 1 to 12 is applied, **characterized by** comprising following steps:
S 1-performing nucleic acid extraction, wherein the first taking and placing device (200) transfers the sample tube (900) on the sample rack (500) to the first lid opening and closing device (300), so as to open a lid of the sample tube (900) by the first lid opening and closing device, and then, a first pipette tip on the consumable rack (600) is taken such that the first pipette tip sucks a sample in the sample tube (900), and places the sample sucked by the first pipette tip in the nucleic acid purification module for nucleic acid extraction;
S2-performing PCR system construction, wherein the first taking and placing device (200) transfers the PCR tube (1000) on the consumable rack (600) to the centrifugal transfer device (800), a PCR tube lid is opened by the second lid opening and closing device (400), and the first taking and placing device (200) then takes a second pipette tip on the consumable rack (600), allows the second pipette tip to suck a reagent, adds the reagent into the PCR tube (1000), then takes a third pipette tip on the consumable rack (600), allows the third pipette tip to suck a gene sample extracted in the nucleic acid purification module, and adds the gene sample into the PCR tube (1000);
S3-performing centrifugal treatment, wherein the PCR tube lid is closed by the second lid opening and closing device (400) and then, the centrifugal transfer device (800) performs rotary centrifugal treatment on the PCR tube (1000) and transfers the PCR tube (1000) after the centrifugal treatment to the PCR detection region (120); and
S4-performing PCR detection, wherein the second taking and placing device transfers the PCR tube (1000) located in the PCR detection region (120) to the PCR detection device (700) for detection.

## Patentansprüche

1. Vollautomatische Genanalysevorrichtung, **dadurch gekennzeichnet, dass** sie umfasst:
einen Kasten (100), eine erste Aufnahme- und Platzierungsvorrichtung (200), eine zweite Aufnahme- und Platzierungsvorrichtung, eine erste Deckelöffnungs- und - schließvorrichtung (300), ein Probengestell (500), ein Verbrauchsmaterialgestell (600), eine PCR-Nachweisvorrichtung (700), eine Zentrifugenvorrichtung (800) und ein Nukleinsäure-Aufreinigungsmodul (180), wobei
der Kasten (100) einen Nukleinsäure-Aufreinigungsbereich (110), einen PCR-Nachweisbereich (120) und einen PCR-Pufferbereich (130) umfasst;
die erste Aufnahme- und Platzierungsvorrichtung (200), die erste Deckelöffnungs- und - schließvorrichtung (300), das Probengestell (500), das Verbrauchsmaterialgestell (600) und das Nukleinsäure-Aufreinigungsmodul (180) alle in dem Nukleinsäure-Aufreinigungsbereich (110) angebracht sind, und die erste Aufnahme- und Platzierungsvorrichtung (200) konfiguriert ist, um bewegt zu werden, das Probengestell (500), das Verbrauchsmaterialgestell (600), die erste Deckelöffnungs- und - schließvorrichtung (300) und die Zentrifugenvorrichtung (800) und ein Probenröhrchen (900) oder ein PCR-Röhrchen (1000) aufzunehmen und zu platzieren oder einen Pipettiervorgang durchzuführen;
die PCR-Nachweisvorrichtung (700) und die zweite Aufnahme- und Platzierungsvorrichtung beide in dem PCR-Nachweisbereich (120) angebracht sind und die zweite Aufnahme- und Platzierungsvorrichtung so konfiguriert ist, dass sie zu der PCR-Nachweisvorrichtung (700) bewegt werden kann und das PCR-Röhrchen (1000) aufnehmen und platzieren kann; und
die Zentrifugalvorrichtung (800) in dem PCR-Pufferbereich (130) angebracht ist; **dadurch gekennzeichnet, dass**
die vollautomatische Genanalysevorrichtung ferner eine zweite Deckelöffnungs- und - schließvorrichtung umfasst, die in dem PCR-Pufferbereich (130) angebracht ist;
die Zentrifugalvorrichtung (800) eine Zentrifugaltransfervorrichtung ist, wobei ein Teil der Zentrifugaltransfervorrichtung (800) im Nukleinsäure- Aufreinigungsbereich (110) angeordnet ist, der andere Teil der Zentrifugaltransfervorrichtung im PCR-Nachweisbereich (120) angeordnet ist, und die Zentrifugaltransfervorrichtung (800) so konfiguriert ist, dass sie gedreht werden kann, um das PCR-Röhrchen (1000) auf der Zentrifugaltransfervorrichtung anzutreiben, um sich in den Nukleinsäure-Aufreinigungsbereich (110) oder den PCR-Nachweisbereich (120) zu bewegen;
die erste Aufnahme- und Platzierungsvorrichtung (200) so konfiguriert ist, dass sie zu dem Nukleinsäure- Aufreinigungsmodul (180) bewegt wird; und
die zweite Aufnahme- und Platzierungsvorrichtung so konfiguriert ist, dass sie zu der Zentrifugentransfervorrichtung (800) bewegt wird;
**und dass** die Zentrifugaltransfervorrichtung (800) einen Drehteller (810) und ein Drehantriebselement (3) umfasst, wobei
der Drehteller (810) so konfiguriert ist, dass er das PCR-Röhrchen (1000) anbringt, die zweite Deckelöffnungs- und -schließvorrichtung (400) einen Greifabschnitt (7) aufweist und der Greifabschnitt (7) so konfiguriert ist, dass er einen Röhrchendeckel auf dem PCR-Röhrchen (1000) bewegt; und
das Drehantriebselement (3) in Übertragungsverbindung mit dem Drehteller (810) steht, und das Drehantriebselement (3) einen Drehmodus und einen Zentrifugalmodus aufweist, wobei das Drehantriebselement (3), wenn es sich im Drehmodus befindet, das PCR-Röhrchen (1000) antreibt, um sich in eine Position unterhalb des Greifabschnitts (7) zu bewegen; und , wenn sich das Drehantriebselement (3) im Zentrifugalmodus befindet, das PCR-Röhrchen (1000) antreibt, um eine Zentrifugalbewegung auszuführen, wobei
die Zentrifugaltransfervorrichtung (800) ferner Montagebaugruppen (820) umfasst, wobei
die PCR-Röhrchen (1000) in den Montagebaugruppen (820) angeordnet sind, mehrere Montagebaugruppen (820) vorgesehen sind, die mehreren Montagebaugruppen (820) gleichmäßig entlang einer äußeren Umfangsrichtung des Drehtellers (810) angeordnet sind, und die Montagebaugruppen (820) an dem Drehteller (810) angelenkt sind; und
zwischen zwei beliebigen benachbarten Montagebaugruppen (820) ein Röhrchendeckelplatzierungsabschnitt (6) vorgesehen ist;
die zweite Deckelöffnungs- und -schließvorrichtung (400) ein Greiferantriebselement (8), eine Verbindungsschraube (9), eine Mutterhülse (10), einen beweglichen Sitz (11) und einen Keil (12) umfasst, wobei
das Greiferantriebselement (8) in Übertragungsverbindung mit der Verbindungsschraube (9) steht, die Mutterhülse (10) auf der Verbindungsschraube (9) vorgesehen ist, die Mutterhülse (10) mit dem Keil (12) durch den beweglichen Sitz (11) verbunden ist, und das Greiferantriebselement (8) so konfiguriert ist, dass es die Verbindungsschraube (9) antreibt, um sich in einer Achsenrichtung der Verbindungsschraube zu drehen, um so die Mutterhülse (10), den beweglichen Sitz (11) und den Keil (12) in der Achsenrichtung der Verbindungsschraube (9) zu bewegen; und
der Keil (12) eine geneigte Fläche aufweist, der Greifabschnitt (7) gegen die geneigte Fläche stößt und der Keil (12) sich bewegt, um den Greifabschnitt (7) anzutreiben, um den Röhrchendeckel zu klemmen oder zu lösen; und dass
die zweite Deckelöffnungs- und -schließvorrichtung (400) ferner ein Hebeantriebselement (13), eine Befestigungsmontageplatte (14) und einen Befestigungssitz (15) umfasst, wobei der Befestigungssitz (15) mit der Befestigungsmontageplatte (14) verbunden ist, eine Gleitrille in dem Befestigungssitz (15) vorgesehen ist, die Verbindungsschraube (9) durch den Befestigungssitz (15) in die Gleitrille eindringt und die Mutterhülse (10) in der Gleitrille angeordnet ist; und
das Hebe-Antriebselement (13) mit der Befestigungs-Montageplatte (14) verbunden ist, und das Hebe-Antriebselement (13) so konfiguriert ist, dass es die Befestigungs-Montageplatte (14) antreibt, sich zu bewegen, um den Greifabschnitt (7) zu dem PCR- Röhrchen (1000) hin oder von ihm weg zu bewegen.

2. Die vollautomatische Genanalysevorrichtung nach Anspruch 1, wobei der PCR-Pufferbereich (130) über dem Nukleinsäure- Aufreinigungsbereich (110) und dem PCR-Nachweisbereich (120) vorgesehen ist, der PCR-Pufferbereich (130) eine abgedichtete Kammer ist, und die abgedichtete Kammer mit einer ersten Öffnung (131), die sich in dem Nukleinsäure- Aufreinigungsbereich (110) befindet, und einer zweiten Öffnung (132), die sich in dem PCR-Nachweisbereich (120) befindet, versehen ist, wobei
die erste Öffnung (131) mit einer ersten Tür zum Öffnen und Schließen der ersten Öffnung versehen ist, und die zweite Öffnung (132) mit einer zweiten Tür zum Öffnen und Schließen der zweiten Öffnung versehen ist; und
die Zentrifugaltransfervorrichtung (800) und die zweite Deckelöffnungs- und - schließvorrichtung (400) beide innerhalb der abgedichteten Kammer und unterhalb der ersten Öffnung (131) und der zweiten Öffnung (132) angeordnet sind.

3. Die vollautomatische Genanalysevorrichtung nach Anspruch 2, die ferner eine Unterdruckabsaugvorrichtung (1100) und eine Luftbehandlungsvorrichtung (1200) umfasst, wobei die Unterdruckabsaugvorrichtung (1100) jeweils mit dem PCR-Nachweisbereich (120) und der Luftbehandlungsvorrichtung (1200) in Verbindung steht und die Luftbehandlungsvorrichtung (1200) mit der Außenatmosphäre in Verbindung steht, und die Unterdruck-Saugvorrichtung so konfiguriert ist, dass sie einen Unterdruckeffekt erzeugt, so dass der Gasdruck des Nukleinsäure-Aufreinigungsbereichs größer ist als der des PCR-Pufferbereichs, und der Gasdruck des PCR-Pufferbereichs größer ist als der des PCR-Nachweisbereichs.

4. Die vollautomatische Genanalysevorrichtung nach Anspruch 3, wobei die Luftbehandlungsvorrichtung (1200) einen Luftfilter (1210), einen Reagenzlösungsfilter (1220) und ein Abluftrohr (1230) umfasst, und die Unterdruck- Absaugvorrichtung (1100), der Luftfilter (1210), der Reagenzlösungsfilter (1220) und das Abluftrohr (1230) aufeinanderfolgend miteinander verbunden sind.

5. Die vollautomatische Genanalysevorrichtung nach Anspruch 2, wobei ein Einwegventil zwischen der abgedichteten Kammer und dem PCR-Nachweisbereich (120) vorgesehen ist, um einen unidirektionalen Gasströmungsfluss von der abgedichteten Kammer zum PCR-Nachweisbereich zu ermöglichen.

6. Die vollautomatische Genanalysevorrichtung nach einem der Ansprüche 1 bis 5, wobei die erste Aufnahme- und Platzierungsvorrichtung (200) einen Bewegungsmechanismus (210) und einen ersten Klemmmechanismus (220) umfasst, die miteinander verbunden sind, und der Bewegungsmechanismus (210) so konfiguriert ist, dass er den ersten Klemmmechanismus (220) zum Verschieben und Heben und Senken antreibt, und
der erste Klemmmechanismus (220) zwei Klemmarme (230) umfasst, wobei jeder Klemmarm (230) mit einer ersten Klemmstruktur (231) und einer zweiten Klemmstruktur (232) versehen ist, wobei zwei erste Klemmstrukturen (231) eingepasst sind, um einen ersten Klemmabschnitt zu bilden, und zwei zweite Klemmstrukturen (232) eingepasst sind, um einen zweiten Klemmabschnitt zu bilden,
die erste Klemmstruktur (231) eine Bogennut ist, die in einer Innenseite des Klemmarms (230) ausgebildet ist, und/oder die zweite Klemmstruktur (232) ein Klemmblock ist, der an einem unteren Ende des Klemmarms (230) vorgesehen ist;
die erste Aufnahme- und Platzierungsvorrichtung (200) ferner eine Pipettiervorrichtung umfasst, die so konfiguriert ist, dass sie einen Pipettiervorgang durchführt, nachdem eine Pipettenspitze aufgenommen wurde.

7. Die vollautomatische Genanalysevorrichtung nach einem der Ansprüche 1 bis 5, wobei die PCR-Nachweisvorrichtung (700) eine Vielzahl von PCR-Nachweismechanismen umfasst, die Vielzahl von PCR-Nachweismechanismen sequentiell in einer voreingestellten Richtung angeordnet sind, die zweite Aufnahme- und Platzierungsvorrichtung so konfiguriert ist, dass sie sequentiell PCR-Röhrchen (1000) in die Vielzahl von PCR-Nachweismechanismen platziert, wobei die vollautomatische Genanalysevorrichtung so konfiguriert ist, dass sie den Nachweis durchführt, nachdem alle Lochpositionen der PCR-Nachweismechanismen mit den PCR-Gefäßen bestückt worden sind.

8. Die vollautomatische Genanalysevorrichtung nach einem der Ansprüche 1 bis 5, wobei die Zentrifugaltransfervorrichtung (800) ferner eine Röhrchenkörperbefestigungsbaugruppe (16) umfasst;
eine Motorgrundplatte (17) an einem Boden des Drehantriebselements (3) vorgesehen ist, eine Befestigungsgrundplatte (18) auf einer von dem Drehantriebselement (3) abgewandten Seite der Motorgrundplatte (17) vorgesehen ist, und die zweite Deckelöffnungs- und - schließvorrichtung (400) und die Röhrchenkörperbefestigungsbaugruppe (16) beide auf der Befestigungsgrundplatte (18) vorgesehen sind; und
die Röhrchenkörperbefestigungsbaugruppe (16) eine Röhrchenkörperklemme (19) aufweist, die zum Klemmen des PCR- Röhrchens (1000) konfiguriert ist,
die Röhrchenkörperbefestigungsbaugruppe (16) ein Klemm- Antriebselement (20), eine Klemm- Schraube (21) und eine Klemm- Mutter (22) aufweist,
wobei das Klemm- Antriebselement (20) in Übertragungsverbindung mit der KlemmSchraube (21) steht, die Klemmmutter (22) auf der Klemmschraube (21) vorgesehen ist, die Klemmmutter (22) mit der Röhrchenkörperklemme (19) verbunden ist und das Klemmantriebselement (20) so konfiguriert ist, dass es die Klemmschraube (21) so antreibt, dass sie sich in einer Achsenrichtung der Klemmschraube dreht, um die Röhrche Röhrchenkörperklemme (19) zu dem PCR-Röhrchen (1000) hin oder von ihm weg zu bewegen.

9. Die vollautomatische Genanalysevorrichtung nach Anspruch 8, wobei
die Röhrchenkörperbefestigungsbaugruppe (16) ferner einen Klemmhalterungsrahmen (23) umfasst,
wobei das Klemmantriebselement (20) mit dem Klemmhalterungsrahmen (23) verbunden ist und der Klemmhalterungsrahmen (23) mit einem Begrenzungssignalsensor (24) und einem Ursprungssignalsensor (25) versehen ist.

10. Die vollautomatische Genanalysevorrichtung nach Anspruch 8, wobei
die Zentrifugaltransfervorrichtung (800) weiterhin ein Außengehäuse (26), eine obere Abdeckplatte (27) und eine hintere Abdeckplatte (28) umfasst,
wobei die obere Abdeckplatte (27) und die hintere Abdeckplatte (28) beide mit dem Außengehäuse (26) verbunden sind, die Befestigungsgrundplatte (18) jeweils mit dem äußeren Gehäuse (26) und der hinteren Abdeckplatte (28) verbunden ist, die Befestigungsgrundplatte (18), die obere Abdeckplatte (27), die hintere Abdeckplatte (28) und das äußere Gehäuse (26) einen Aufnahmehohlraum bilden, und die zweite Deckelöffnungs- und -schließvorrichtung (400) in dem Aufnahmehohlraum angeordnet ist,
zwischen dem Außengehäuse (26) und der oberen Abdeckplatte (27) eine obere Deckeldichtungsleiste (29) vorgesehen ist;
die Befestigungsgrundplatte (18) mit einer unteren Dichtleiste (30) versehen ist;
zwischen der hinteren Abdeckplatte (28) und dem Außengehäuse (26) eine erste Dichtleiste (31) vorgesehen ist; und
ein innerer Rahmen (32) in dem Aufnahmehohlraum vorgesehen ist und ein zweiter Dichtungsstreifen (33) zwischen dem inneren Rahmen (32) und dem äußeren Gehäuse (26) vorgesehen ist;
die Zentrifugaltransfervorrichtung (800) ferner eine Türöffnungs- und -schließanordnung (35) umfasst; und
die obere Abdeckplatte (27) mit einer Öffnung versehen ist, die Türöffnungs- und - schließanordnung (35) eine Türdrückplatte (36) aufweist, die Öffnung durch die Türdrückplatte (36) verschlossen werden kann, und ein Türplattendichtungsstreifen (34) zwischen der Türöffnungs- und -schließanordnung (35) und der oberen Abdeckplatte (27) vorgesehen ist.

11. Die vollautomatische Genanalysevorrichtung nach Anspruch 10, wobei
die Türöffnungs- und -schließanordnung (35) einen Linearmotor (37), einen Türrahmen (38), eine erste Federaufhängung (39), eine zweite Federaufhängung (40) und ein elastisches Element (41) umfasst, wobei
der Linearmotor (37) an der oberen Abdeckplatte (27) vorgesehen ist, der Linearmotor (37) in Übertragungsverbindung mit dem Türrahmen (38) steht, die erste Federaufhängung (39) an dem Türrahmen (38) vorgesehen ist, ein nierenförmiges Loch (42) in dem Türrahmen (38) vorgesehen ist und die Türdrückplatte (36) sich in das nierenförmige Loch (42) durch die zweite Federaufhängung (40) erstreckt; und
ein Ende des elastischen Elements (41) mit der ersten Federaufhängung (39) verbunden ist, das andere Ende des elastischen Elements (41) mit der zweiten Federaufhängung (40) verbunden ist und das elastische Element (41) so konfiguriert ist, dass es der Türandrückplatte (36) eine Tendenz verleiht, sich in Richtung des Türrahmens (38) zu bewegen.

12. Die vollautomatische Genanalysevorrichtung nach Anspruch 11, wobei
die obere Abdeckplatte (27) mit einem Begrenzungsblock (43) versehen ist; und
der Linearmotor (37) den Türrahmen (38) antreibt, um sich in Richtung des Begrenzungsblocks (43) zu bewegen, so dass die Türdrückplatte (36) gegen den Begrenzungsblock (43) stößt und die Türdruckplatte (36) sich entlang des nierenförmigen Lochs (42) bewegt, um den Türplattendichtungsstreifen (34) fest zu drücken.

13. Ein Genanalyseverfahren, bei dem die vollautomatische Genanalysevorrichtung nach einem der Ansprüche 1 bis 12 angewendet wird, **dadurch gekennzeichnet, dass** es folgende Schritte umfasst::
S1-Durchführen einer Nukleinsäureextraktion, wobei die erste Aufnahme- und Platzierungsvorrichtung (200) das Probenröhrchen (900) auf dem Probengestell (500) an die erste Deckelöffnungs- und Schließvorrichtung (300) übergibt, um einen Deckel des Probenröhrchens (900) durch die erste Deckelöffnungs- und Schließvorrichtung zu öffnen, und dann eine erste Pipettenspitze auf dem Verbrauchsmaterial-Rack (600) genommen wird, so dass die erste Pipettenspitze eine Probe in dem Probenröhrchen (900) ansaugt, und die von der ersten Pipettenspitze angesaugte Probe in das Nukleinsäure- Aufreinigungsmodul zur Nukleinsäure-Extraktion platziert;
S2- Durchführen eines PCR-Systemaufbaus, wobei die erste Aufnahme- und Platzierungsvorrichtung (200) das PCR- Röhrchen (1000) auf dem Verbrauchsmaterialgestell (600) an die Zentrifugaltransfervorrichtung (800) übergibt, ein PCR- Röhrchendeckel durch die zweite Deckelöffnungs- und -schließvorrichtung (400) geöffnet wird, und die erste Aufnahme- und Platzierungsvorrichtung (200) dann eine zweite Pipettenspitze auf dem Verbrauchsmaterialgestell (600) aufnimmt, die zweite Pipettenspitze ein Reagenz ansaugen lässt, das Reagenz in das PCR-Röhrchen (1000) gibt, dann eine dritte Pipettenspitze auf dem Verbrauchsmaterialgestell (600) aufnimmt, die dritte Pipettenspitze eine Genprobe ansaugen lässt, die in dem Nukleinsäure-Aufreinigungsmodul extrahiert wurde, und die Genprobe in das PCR-Röhrchen (1000) gibt;
S3-Durchführen einer Zentrifugalbehandlung, wobei der PCR-Röhrchendeckel durch die zweite Deckelöffnungs- und -schließvorrichtung (400) verschlossen wird und dann die Zentrifugaltransfervorrichtung (800) eine Rotationszentrifugalbehandlung an dem PCR-Röhrchen (1000) durchführt und das PCR-Röhrchen (1000) nach der Zentrifugalbehandlung in den PCR-Nachweisbereich (120) transferiert; und
S4-Durchführen eines PCR-Nachweises, wobei die zweite Aufnahme- und Platzierungsvorrichtung das PCR-Röhrchen (1000), das sich in dem PCR-Nachweisbereich (120) befindet, zu der PCR-Nachweisvorrichtung (700) zum Nachweis transferiert.

## Revendications

1. Appareil d'analyse génétique entièrement automatique, **caractérisé en ce qu'**il comprend :
un corps (100), un premier dispositif de prise et de mise en place (200), un second dispositif de prise et de mise en place, un premier dispositif d'ouverture et de fermeture du couvercle (300), un rack d'échantillons (500), un rack de consommables (600), un dispositif de détection PCR (700), un dispositif centrifuge (800) et un module de purification de l'acide nucléique (180), dans lequel
le corps (100) comprend une région de purification de l'acide nucléique (110), une région de détection PCR (120) et une région de tampons PCR (130);
le premier dispositif de prise et de mise en place (200), le premier dispositif d'ouverture et de fermeture du couvercle (300), le rack d'échantillons (500), le rack de consommables (600) et le module de purification de l'acide nucléique (180) sont tous montés dans la région de purification de l'acide nucléique (110), et le premier dispositif de prise et de mise en place (200) est configuré pour être déplacé, le rack d'échantillons (500), le rack de consommables (600), le premier dispositif d'ouverture et de fermeture du couvercle (300) et le dispositif centrifuge (800), et pour prendre et mettre en place un tube d'échantillon (900) ou un tube PCR (1000) ou pour effectuer une opération de pipetage;
le dispositif de détection PCR (700) et le second dispositif de prise et de mise en place sont tous deux montés dans la région de détection PCR (120), et le second dispositif de prise et de mise en place est configuré pour être déplacé vers le dispositif de détection PCR (700) et pour prendre et mettre en place le tube PCR (1000); et
le dispositif centrifuge (800) est monté dans la région tampon PCR (130); **caractérisé en ce que**
l'appareil d'analyse génétique entièrement automatique comprend en outre un deuxième dispositif d'ouverture et de fermeture du couvercle qui est monté dans la zone tampon PCR (130);
le dispositif centrifuge (800) est un dispositif de transfert centrifuge dans lequel une partie du dispositif de transfert centrifuge (800) est située dans la région de purification de l'acide nucléique (110), l'autre partie du dispositif de transfert centrifuge est située dans la région de détection PCR (120), et le dispositif de transfert centrifuge (800) est configuré pour être tourné afin d'entraîner le tube PCR (1000) sur le dispositif de transfert centrifuge pour se déplacer dans la région de purification de l'acide nucléique (110) ou dans la région de détection PCR (120);
le premier dispositif de prise et de mise en place (200) est configuré pour être déplacé vers le module de purification de l'acide nucléique (180); et
le second dispositif de prise et de mise en place est configuré pour être déplacé vers le dispositif de transfert centrifuge (800);
et **en ce que** le dispositif de transfert centrifuge (800) comprend un disque rotatif (810) et un élément d'entraînement rotatif (3), dans lequel
le disque rotatif (810) est configuré pour monter le tube PCR (1000), le second dispositif d'ouverture et de fermeture de couvercle (400) a une partie de saisie (7), et la partie de saisie (7) est configurée pour entraîner le déplacement d'un couvercle de tube sur le tube PCR (1000); et
l'élément d'entraînement rotatif (3) est en liaison de transmission avec le disque rotatif (810), et l'élément d'entraînement rotatif (3) a un mode rotatif et un mode centrifuge, dans lequel
en mode rotatif, l'élément d'entraînement rotatif (3) entraîne le tube PCR (1000) à se déplacer vers une position en dessous de la partie de saisie (7); et
en mode centrifuge, l'élément d'entraînement rotatif (3) entraîne le tube PCR (1000) pour effectuer un mouvement centrifuge,
le dispositif de transfert centrifuge (800) comprend en outre des ensembles de montage (820), où
les tubes PCR (1000) sont placés dans les assemblages de montage (820), plusieurs assemblages de montage (820) sont prévus, les plusieurs assemblages de montage (820) sont disposés uniformément le long d'une direction de circonférence extérieure du disque rotatif (810), et les assemblages de montage (820) sont articulés sur le disque rotatif (810); et
une partie de placement de couvercle de tube (6) est prévue entre deux ensembles de montage adjacents (820) ;
le second dispositif d'ouverture et de fermeture de couvercle (400) comprend un élément d'entraînement de pince (8), une vis de connexion (9), un manchon d'écrou (10), un siège mobile (11) et une cale (12), où
l'élément d'entraînement de la pince (8) est en liaison de transmission avec la vis de connexion (9), le manchon d'écrou (10) est prévu sur la vis de connexion (9), le manchon d'écrou (10) est relié à la cale (12) par le siège mobile (11), et l'élément d'entraînement de la pince (8) est configuré pour entraîner la vis de connexion (9) à tourner dans une direction d'axe de la vis de connexion, de manière à déplacer le manchon d'écrou (10), le siège mobile (11) et la cale (12) dans la direction d'axe de la vis de connexion (9); et
la cale (12) a une surface inclinée, la partie saisissante (7) est en contact avec la surface inclinée, et la cale (12) se déplace pour entraîner la partie saisissante (7) afin de serrer ou de relâcher le couvercle du tube; **et en ce que**
le deuxième dispositif d'ouverture et de fermeture de couvercle (400) comprend en outre un élément moteur de levage (13), une plaque de montage de fixation (14) et un siège de fixation (15), où
le siège de fixation (15) est relié à la plaque de montage de fixation (14), une rainure de glissement est prévue dans le siège de fixation (15), la vis de connexion (9) pénètre à travers le siège de fixation (15) dans la rainure de glissement, et le manchon d'écrou (10) est située dans la rainure de glissement; et
l'élément moteur de levage (13) est relié à la plaque de montage de fixation (14), et l'élément moteur de levage (13) est configuré pour entraîner le déplacement de la plaque de montage de fixation (14), de manière à rapprocher ou à éloigner la partie saisissante (7) du tube PCR (1000).

2. L'appareil d'analyse génétique entièrement automatique selon la revendication 1, dans lequel la région tampon PCR (130) est prévue à travers la région de purification de l'acide nucléique (110) et la région de détection PCR (120), la région tampon PCR (130) est une chambre scellée, et la chambre scellée est pourvue d'une première ouverture (131) située dans la région de purification de l'acide nucléique (110) et d'une seconde ouverture (132) située dans la région de détection PCR (120), où
la première ouverture (131) est pourvue d'une première porte permettant d'ouvrir et de fermer la première ouverture, et la seconde ouverture (132) est pourvue d'une seconde porte permettant d'ouvrir et de fermer la seconde ouverture; et
le dispositif de transfert centrifuge (800) et le second dispositif d'ouverture et de fermeture du couvercle (400) sont tous deux situés à l'intérieur de la chambre scellée et sous la première ouverture (131) et la seconde ouverture (132).

3. L'appareil d'analyse génétique entièrement automatique selon la revendication 2, comprenant en outre un dispositif d'aspiration à pression négative (1100) et un dispositif de traitement de l'air (1200), dans lequel le dispositif d'aspiration à pression négative (1100) est respectivement en communication avec la région de détection PCR (120) et le dispositif de traitement de l'air (1200), le dispositif de traitement de l'air (1200) étant en communication avec l'atmosphère extérieure, et le dispositif d'aspiration à pression négative est configuré pour produire un effet de pression négative tel qu'une pression de gaz de la région de purification de l'acide nucléique est supérieure à celle de la région tampon PCR, et qu'une pression de gaz de la région tampon PCR est supérieure à celle de la région de détection PCR.

4. L'appareil d'analyse génétique entièrement automatique selon la revendication 3, dans lequel le dispositif de traitement de l'air (1200) comprend un filtre de l'air (1210), un filtre de la solution de réactif (1220) et un tuyau d'échappement (1230), et le dispositif d'aspiration à pression négative (1100), le filtre de l'air (1210), le filtre de la solution de réactif (1220) et le tuyau d'échappement (1230) sont communiqués de manière séquentielle.

5. Appareil d'analyse génétique entièrement automatique selon la revendication 2, dans lequel une vanne à sens unique est prévue entre la chambre scellée et la région de détection PCR (120) pour permettre un flux de courant gazeux unidirectionnel depuis la chambre scellée jusqu'à la zone de détection PCR.

6. L'appareil d'analyse génétique entièrement automatique selon l'une quelconque des revendications 1 à 5, dans lequel le premier dispositif de prise et de mise en place (200) comprend un mécanisme de mouvement (210) et un premier mécanisme de serrage (220) reliés l'un à l'autre, et le mécanisme de mouvement (210) est configuré pour entraîner le premier mécanisme de serrage (220) à se déplacer et à se soulever et à s'abaisser; et
le premier mécanisme de serrage (220) comprend deux bras de serrage (230), chaque bras de serrage (230) est pourvu d'une première structure de serrage (231) et d'une seconde structure de serrage (232), où deux premières structures de serrage (231) sont ajustées pour former une première partie de serrage, et deux secondes structures de serrage (232) sont ajustées pour former une seconde partie de serrage,
la première structure de serrage (231) est une rainure en arc formée dans un côté intérieur du bras de serrage (230), et/ou la seconde structure de serrage (232) est un bloc de serrage fourni à une extrémité inférieure du bras de serrage (230);
le premier dispositif de prise et de mise en place (200) comprend en outre un dispositif de pipetage configuré pour effectuer une opération de pipetage après la prise d'une pointe de pipette.

7. L'appareil d'analyse génétique entièrement automatique selon l'une quelconque des revendications 1 à 5, dans lequel le dispositif de détection PCR (700) comprend une pluralité de mécanismes de détection PCR, la pluralité de mécanismes de détection PCR sont disposés séquentiellement dans une direction prédéfinie, le second dispositif de prise et de mise en place est configuré pour placer séquentiellement des tubes PCR (1000) dans la pluralité de mécanismes de détection PCR, l'appareil d'analyse génétique entièrement automatique est configuré pour effectuer la détection après que toutes les positions des trous des mécanismes de détection PCR ont été chargées avec les tubes PCR.

8. L'appareil d'analyse génétique entièrement automatique selon l'une quelconque des revendications 1 à 5, dans lequel
le dispositif de transfert centrifuge (800) comprend en outre un ensemble de fixation du corps du tube (16) ;
une plaque de base du moteur (17) est prévue au bas de l'élément d'entraînement rotatif (3), une plaque de base de fixation (18) est prévue sur un côté de la plaque de base du moteur (17) à l'écart de l'élément d'entraînement rotatif (3), et le deuxième dispositif d'ouverture et de fermeture du couvercle (400) et l'ensemble de fixation du corps du tube (16) sont tous deux prévus sur la plaque de base de fixation (18); et
l'ensemble de fixation du corps du tube (16) a une pince du corps du tube (19) configurée pour serrer le tube PCR (1000),
l'ensemble de fixation du corps du tube (16) comprend un élément moteur de serrage (20), une vis de serrage (21) et un écrou de serrage (22),
dans lequel l'élément moteur de serrage (20) est en liaison de transmission avec la vis de serrage (21), l'écrou de serrage (22) est prévu sur la vis de serrage (21), l'écrou de serrage (22) est relié à la pince du corps du tube (19), et l'élément moteur de serrage (20) est configuré pour entraîner la vis de serrage (21) à tourner dans une direction d'axe de la vis de serrage, de manière à déplacer la pince du corps du tube (19) vers ou à l'écart du tube PCR (1000).

9. L'appareil d'analyse génétique entièrement automatique selon la revendication 8, dans lequel
l'ensemble de fixation du corps du tube (16) comprend en outre un cadre de montage de serrage (23),
dans lequel l'élément moteur de serrage (20) est relié au cadre de montage de serrage (23), et le cadre de montage de serrage (23) est pourvu d'un capteur de signal de limitation (24) et d'un capteur de signal d'origine (25).

10. L'appareil d'analyse génétique entièrement automatique selon la revendication 8, dans lequel
le dispositif de transfert centrifuge (800) comprend en outre un boîtier extérieur (26), une plaque de couverture supérieure (27) et une plaque de couverture arrière (28),
où la plaque de couverture supérieure (27) et la plaque de couverture arrière (28) sont toutes deux reliées au boîtier extérieur (26), la plaque de base de fixation (18) est respectivement reliée au boîtier extérieur (26) et à la plaque de couverture arrière (28), la plaque de base de fixation (18), la plaque de couverture supérieure (27), la plaque de couverture arrière (28) et le boîtier extérieur (26) forment une cavité de réception, et le second dispositif d'ouverture et de fermeture du couvercle (400) est situé dans la cavité de réception,
une bande d'étanchéité de couvercle supérieur (29) est prévue entre le boîtier extérieur (26) et la plaque de couverture supérieure (27) ;
la plaque de base de fixation (18) est pourvue d'une bande d'étanchéité inférieure (30);
une première bande d'étanchéité (31) est prévue entre la plaque de couverture arrière (28) et le boîtier extérieur (26); et
un cadre intérieur (32) est prévu dans la cavité de réception, et une deuxième bande d'étanchéité (33) est prévue entre le cadre intérieur (32) et le boîtier extérieur (26);
le dispositif de transfert centrifuge (800) comprend en outre un ensemble d'ouverture et de fermeture de porte (35); et
la plaque de couverture supérieure (27) est pourvue d'une ouverture, l'ensemble d'ouverture et de fermeture de porte (35) a une plaque de pression de porte (36), l'ouverture peut être bouchée par la plaque de pression de porte (36), et une bande d'étanchéité de plaque de porte (34) est prévue entre l'ensemble d'ouverture et de fermeture de porte (35) et la plaque de couverture supérieure (27).

11. L'appareil d'analyse génétique entièrement automatique selon la revendication 10, dans lequel
l'ensemble d'ouverture et de fermeture de porte (35) comprend un moteur linéaire (37), un cadre de porte (38), un premier suspension à ressort (39), un second suspension à ressort (40) et un élément élastique (41), dans lequel
le moteur linéaire (37) est prévu sur la plaque de couverture supérieure (27), le moteur linéaire (37) est en liaison de transmission avec le cadre de la porte (38), la première suspension à ressort (39) est prévue sur le cadre de la porte (38), un trou en forme de rein (42) est prévu dans le cadre de la porte (38), et la plaque de pression de la porte (36) s'étend dans le trou en forme de rein (42) à travers la deuxième suspension à ressort (40); et
une extrémité de l'élément élastique (41) est reliée à la première suspension à ressort (39), l'autre extrémité de l'élément élastique (41) est reliée à la deuxième suspension à ressort (40),
et l'élément élastique (41) est configuré pour donner à la plaque de pression de porte (36) une tendance à se déplacer vers le cadre de porte (38).

12. L'appareil d'analyse génétique entièrement automatique selon la revendication 11, dans lequel
la plaque de couverture supérieure (27) est pourvue d'un bloc de limitation (43); et
le moteur linéaire (37) entraîne le cadre de la porte (38) à se déplacer vers le bloc de limitation (43), de sorte que la plaque de pression de la porte (36) bute contre le bloc de limitation (43), et la plaque de pression de la porte (36) se déplace le long du trou en forme de rein (42) pour presser fermement la bande d'étanchéité de la plaque de la porte (34).

13. Une méthode d'analyse génétique, à laquelle est appliqué l'appareil d'analyse génétique entièrement automatique selon l'une quelconque des revendications 1 à 12, **caractérisée par** les étapes suivantes :
S1-réaliser une extraction d'acide nucléique, dans laquelle le premier dispositif de prise et de mise en place (200) transfère le tube d'échantillon (900) sur le rack d'échantillon (500) au premier dispositif d'ouverture et de fermeture du couvercle (300), de manière à ouvrir un couvercle du tube d'échantillon (900) par le premier dispositif d'ouverture et de fermeture du couvercle, et puis, une première pointe de pipette sur le rack de consommables (600) est prise de telle sorte que la première pointe de pipette aspire un échantillon dans le tube d'échantillon (900), et place l'échantillon aspiré par la première pointe de pipette dans le module de purification de l'acide nucléique pour l'extraction de l'acide nucléique;
S2-performance de la construction du système PCR, dans lequel le premier dispositif de prise et de mise en place (200) transfère le tube PCR (1000) sur le support de consommables (600) au dispositif de transfert centrifuge (800), un couvercle de tube PCR est ouvert par le second dispositif d'ouverture et de fermeture de couvercle (400), et le premier dispositif de prise et de mise en place (200) prend ensuite une deuxième pointe de pipette sur le support de consommables (600), permet à la deuxième pointe de pipette d'aspirer un réactif, ajoute le réactif dans le tube PCR (1000), puis prend une troisième pointe de pipette sur le support de consommables (600), permet à la troisième pointe de pipette d'aspirer un échantillon de gène extrait dans le module de purification de l'acide nucléique, et ajoute l'échantillon de gène dans le tube PCR (1000);
S3 - effectuer un traitement centrifuge, dans lequel le couvercle du tube PCR est fermé par le second dispositif d'ouverture et de fermeture du couvercle (400), puis le dispositif de transfert centrifuge (800) effectue un traitement centrifuge rotatif sur le tube PCR (1000) et transfère le tube PCR (1000) après le traitement centrifuge vers la région de détection PCR (120); et
S4 - effectuer une détection PCR, le second dispositif de prise et de placement transférant le tube PCR (1000) situé dans la région de détection PCR (120) au dispositif de détection PCR (700) pour la détection.
